(19)

Europäisches
Patentamt
European
Patent Office
Office européen
des brevets

(11) **EP 2 794 098 B1**

(12) **EUROPÄISCHE PATENTSCHRIFT**

(45) Veröffentlichungstag und Bekanntmachung des Hinweises auf die Patenterteilung:
**14.08.2019 Patentblatt 2019/33**

(21) Anmeldenummer: **12859360.5**

(22) Anmeldetag: **20.12.2012**

(51) Int Cl.:
**B01J 23/889** (2006.01)

(86) Internationale Anmeldenummer:
**PCT/IB2012/057513**

(87) Internationale Veröffentlichungsnummer:
**WO 2013/093824 (27.06.2013 Gazette 2013/26)**

(54) **KATALYSATOR FÜR DIE DEHYDRIERUNG VON KOHLENWASSERSTOFFEN**

CATALYST FOR DEHYDROGENATING HYDROCARBONS

CATALYSEUR DE DÉSHYDROGÉNATION D'HYDROCARBURES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **22.12.2011 EP 11195116**

(43) Veröffentlichungstag der Anmeldung:
**29.10.2014 Patentblatt 2014/44**

(60) Teilanmeldung:
**18202477.8 / 3 470 139**

(73) Patentinhaber: **BASF SE**
**67056 Ludwigshafen am Rhein (DE)**

(72) Erfinder:
• **PATCAS, Florina Corina**
  **67065 Ludwigshafen (DE)**
• **HOUSSIN, Christophe**
  **67245 Lambsheim (DE)**
• **DIETERLE, Martin;**
  **67059 Ludwigshafen (DE)**

(74) Vertreter: **BASF IP Association**
**BASF SE**
**G-FLP-C6**
**67056 Ludwigshafen (DE)**

(56) Entgegenhaltungen:
**EP-A1- 0 181 999      EP-A1- 0 502 510**
**WO-A1-03/064032      CN-A- 1 253 855**
**CN-A- 1 470 325**

EP 2 794 098 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft einen Katalysator zur Dehydrierung von Kohlenwasserstoffen auf Basis von Eisenoxid, welcher zudem mindestens eine Kaliumverbindung, mindestens eine Cerverbindung, 0,7 bis 10 Gew.-% mindestens einer Manganverbindung, gerechnet als $MnO_2$, und 10 bis 200 ppm mindestens einer Titanverbindung, gerechnet als $TiO_2$ enthält, sowie ein Verfahren zu dessen Herstellung. Weiterhin betrifft die vorliegende Erfindung ein Verfahren zur katalytischen Dehydrierung von Kohlenwasserstoffen unter Verwendung des erfindungsgemäßen Katalysators.

Im Stand der Technik ist seit langem der Einsatz von Eisenoxid-basierten Dehydrier-Katalysatoren bei der Dehydrierung verschiedener Kohlenwasserstoffe zu den entsprechenden ungesättigten Kohlenwasserstoffen bekannt. Großtechnisch von Bedeutung sind beispielsweise die Dehydrierung von Ethylbenzol zu Styrol, Isopropylbenzol zu alpha-Methylstyrol, Buten zu Butadien oder Isoamylen zu Isopren. Die Herstellung von Styrol durch heterogen katalysierte Dehydrierung von Ethylbenzol in Gegenwart von Wasserdampf ist ein großtechnisch bereits seit Beginn der dreißiger Jahre durchgeführtes Verfahren und hat sich als Syntheseweg für Styrol durchgesetzt. Styrol stellt eines der wichtigsten Monomere der Kunststoffindustrie dar und wird beispielsweise für die Herstellung von Polystyrol, Acrylnitril-Butadien-Styrol-Polymer (ABS) und synthetischem Kautschuk verwendet.

Die im Stand der Technik beschriebenen Eisenoxid-basierten Dehydrier-Katalysatoren sind in der Regel Multikomponenten-Systeme und enthalten im wesentlichen Eisenoxid und eine Alkalimetallverbindung, welche bei der Katalysatorherstellung beispielsweise als Alkalimetalloxid, -carbonat oder -hydroxid eingesetzt wird. Darüberhinaus enthalten diese Katalysatoren in der Regel verschiedene weitere aktive Komponenten (Promotoren), beispielsweise Oxide der Elemente der 5. und 6. Nebengruppe des Periodensystems oder der Seltenen Erden.

Die katalytische Dehydrierung von aliphatischen oder alkylaromatischen Kohlenwasserstoffen wird großtechnisch üblicherweise in Gegenwart von Wasserdampf bei Temperaturen im Bereich von 500 bis 700 °C durchgeführt. In diesen Verfahren werden der Kohlenwasserstoff und der Wasserdampf gemischt und bei höheren Temperaturen über den Eisenoxid-Dehydrierkatalysator geleitet.

Als eine Folge der Koksbildung bei der katalytischen Dehydrierung (beispielsweise von Ethylbenzol zu Styrol) werden im Laufe des Dehydrier-Verfahrens typischerweise die aktiven Zentren des Dehydrier-Katalysators blockiert, und es kommt zu einer allmählichen Deaktivierung des Katalysators. Um diese Deaktivierung zu vermindern wird dem Kohlenwasserstoff Wasserdampf zugesetzt. Durch den Wasserdampf kann der auf der Katalysatoroberfläche entstandenen Koks in-situ vergast werden, wodurch die aktive Katalysatoroberfläche regeneriert werden kann. Daneben hat der Wasserdampf typischerweise noch die folgenden Funktionen: Lieferung der benötigten Reaktionswärme für die endotherme Dehydrierungsreaktion, Verschiebung des Gleichgewichtes zur Produktseite durch Verringerung der partiellen Drücke der Edukte, Aufrechterhaltung des Oxidationszustand des Eisens gegen die reduzierende Wirkung von Wasserstoff und Kohlenwasserstoff.

Generell sind die Stabilität und die Aktivität des Katalysators umso höher, je höher das Verhältnis von (Wasser-)Dampf zu Kohlenwasserstoff (D/KW-Verhältnis) ist. Jedoch ist es aus Sicht des Energiebedarfs und der damit verbundenen Betriebskosten wünschenswert, das Dampf/Kohlenwasserstoff-Verhältnis zu verringern. Ein niedriges Dampf/Kohlenwasserstoff-Verhältnis begünstigt allerdings typischerweise die Verkokung und irreversible Reduzierung des Dehydrier-Katalysators, so dass die Katalysatoraktivität nach relativ kurzer Zeit absinkt. Daneben ist eine gewisse Menge an Wasserdampf normalerweise notwendig, um dem Reaktionssystem die benötigte Energie zuzuführen.

Um ausreichende Standzeiten bei Dehydrier-Verfahren mit niedrigem Dampf/Kohlenwasserstoff-Verhältnis zu gewährleisten, werden an die Katalysatoren besondere Anforderungen gestellt. Bei der katalytischen Dehydrierung von Kohlenwasserstoffen bezeichnet man im Allgemeinen ein molares Dampf/Kohlenwasserstoff-Verhältnis von kleiner oder gleich 7,35 als niedriges D/KW-Verhältnis. Dies entspricht im Falle der Dehydrierung von Ethylbenzol etwa einem Gewichtsverhältnis von Dampf/Kohlenwasserstoff von kleiner oder gleich 1,25.

Im Stand der Technik sind zahlreiche Dehydrier-Katalysatoren auf der Basis von Eisenoxid beschrieben. EP-A 0 181 999 beschreibt Dehydrier-Katalysatoren enthaltend Eisenoxid, Kaliumoxid, Magnesiumoxid und optional weitere Metallverbindungen. Es wird unter anderem die optionale Zugabe von 0 bis 10 Gew.-% einer Verbindung $Me_2O_3$ mit Me = Cr oder Mn beschrieben. Dokument EP-A 0 181 999 offenbart keine Beispiele zu einem Dehydrier-Katalysator enthaltend eine Manganverbindung. Zudem ist die Zugabe von Titan nicht beschrieben. Der in EP-A 0 181 999 beschriebene Katalysator soll sich insbesondere durch eine verbesserte Stabilität gegen Siedewasser auszeichnen.

Das Dokument WO 96/18457 beschreibt ein restrukturiertes Eisenoxid mit speziellen Partikel-Eigenschaften und dessen Einsatz in Dehydrier-Katalysatoren. Das restrukturierte Eisenoxid soll durch Umsetzen eines Eisenoxids mit einem Restrukturierungsagens erhalten werden, wobei das Restrukturierungsagens insbesondere ausgewählt sein kann aus Verbindungen von Molybdän, Kupfer, Calcium, Zink, Kobalt oder Cer. Das Dokument WO 96/18457 beschreibt die Verwendung dieser Katalysatoren bei Dehydrier-Verfahren mit mittleren bis hohen molaren D/KW-Verhältnissen von etwa 10.

Das Dokument EP-B 0 956 899 beschreibt einen Dehydrier-Katalysator enthaltend Eisenoxid, Kaliumoxid, Magnesiumoxid, ein weiteres Metalloxid und mindestens zwei Seltenerdenmetalloxide. Optional können eine Vielzahl von weiteren Promotoren enthalten sein, darunter beispielsweise $MnO_3$. Die Zugabe von 10 bis 200 ppm mindestens einer Titanverbindung ist in EP-B 0 956 899 nicht beschrieben. Das Dokument EP-B 0 956 899 beschreibt zudem die Verwendung der Katalysatoren zur Dehydrierung von alkylaromatischen Verbindungen bei einem Gewichtsverhältnis von Dampf/Kohlenwasserstoff im Bereich von 0,5 bis 2,5.

Das Dokument EP-A 0 502 510 betrifft Dehydrier-Katalysatoren enthaltend Eisenoxid, Kaliumoxid und 0,005 bis 0,95 Gew.-% Titanoxid als wesentliche Bestandteile. Daneben können weitere Promotoren, insbesondere Ceroxid, Molybdänoxid oder Magnesiumoxid enthalten sein. Die Zugabe von Mangan ist nicht beschrieben.

Das Dokument US 4,220,560 beschreibt einen Dehydrier-Katalysator enthaltend einen Eisen-Chrom-Spinell, der noch ein weiteres Metall ausgewählt aus Kobalt, Zink, Mangan und Magnesium enthält. Es wird zudem die Verwendung des Katalysators für die Dehydrierung von Kohlenwasserstoffen bei einem molaren Dampf/Kohlenwasserstoff-Verhältnis von 9 bis 12 beschrieben. Die Zugabe von Titan wird in dem Dokument US 4,220,560 nicht beschrieben.

Das Dokument US 2006/0106267 offenbart einen Katalysator sowie dessen Verwendung zur Herstellung von Styrol bei einem Dampf/Kohlenwasserstoff-Gewichtsverhältnis von kleiner oder gleich 1,35. Der im Dokument US 2006/0106267 beschriebene Katalysator enthält neben Eisenoxid eine Cerverbindung, eine Kaliumverbindung, eine Molybdänverbindung, eine Erdalkalimetallverbindung sowie einen geringen Anteil an Titandioxid. Neben Titandioxid können weitere Promotoren enthalten sein. Die Zugabe von 0,7 bis 10 Gew.-% einer Manganverbindung ist nicht beschrieben. Das Dokument US 2006/0106267 beschreibt, dass ein Styrol-Katalysator mit möglichst geringem Titangehalt die höchste Stabilität aufweisen soll.

Das Dokument WO 99/49966 beschreibt einen Katalysator, der Eisenoxid, Kaliumoxid, eine Magnesiumverbindung und eine Cerverbindung enthält und eine Eisen-Kalium-Phase aufweist, sowie dessen Verwendung bei der Dehydrierung von Ethylbenzol. Es wird kein Katalysator beschrieben, der eine Titanverbindung und 0,7 bis 10 Gew.-% einer Manganverbindung enthält.

Das Dokument CN-A 1470325 beschreibt einen Dehydrier-Katalysator für die Herstellung von Styrol aus Ethylbenzol, wobei der Katalysator Eisenoxid, Kaliumoxid, Ceroxid Molybdänoxid, Magnesiumoxid, Vanadiumoxid, Cobaltoxid, Manganoxid und Titanoxid als wesentliche Bestandteile enthält. Es wird ein Dehydrier-Verfahren bei einem Gewichtsverhältnis von Dampf/Kohlenwasserstoff von 2 und einer Temperatur von 620 °C beschrieben.

Das Dokument CN-A 1 253 855 beschreibt einen Dehydrier-Katalysator basierend auf einem Eisen-Kalium-Cer-Molybdän-System für die Herstellung von aromatischen Olefinen. Der Katalysator enthält 0,05-10 Gew.-% eines Silca-Sols, Silica-Gels oder Metalloxides.

Das Dokument WO 2003/064032 beschreibt einen Dehydrier-Katalysator zu Dehydrierung von alkylaromatischen Verbindungen. Der Katalysator enthält ein speziellen Eisenoxid, welches durch Zersetzung eines Eisenhalogenides hergestellt wird, und 0.07 bis 0.15 mol % einer Lanthanoiden-Verbindung.

Die Zugabe von Mangan zu Dehydrier-Katalysatoren auf der Basis von Eisenoxid ist beispielsweise in den Publikationen Miyakoshi et al. (Appl. Cat. A 216, 2001, S. 137-146) und Kotarba et al. (J. Cat. 221, 2004, S. 650-652) beschrieben. Miyakoshi et al. beschreibt ein Sol-Gel-Verfahren zur Herstellung eines Eisenoxid-Dehydrier-Katalysators, wobei einen $MnFe_2O_4$ Spinell im Katalysator gebildet werden soll. Kotarba et al. beschreibt einen Mangan-dotierten Eisenoxid-Katalysator enthaltend eine aktive $K_2Fe_{22}O_{34}$ Ferritphase, wobei durch die Mangan-Dotierung der Verlust an Kalium aus dem Katalysator vermindert werden soll.

Die Dokument Liao et al. (Cat. Comm. 9, 2008, S. 1817 - 1821) beschreibt den Einfluss der Zugabe von Titandioxid auf die Struktur und die Reaktivität eines Eisenoxid-Dehydrier-Katalysators. Es wird ein Eisen-Kalium-Dehydrier-Katalysator beschrieben, welchem 1.000 bis 15.000 ppm Titandioxid zugesetzt wurden.

Keines der Dokumente offenbart eine positive Wirkung von Mangan in Kombination mit Titan in einem Dehydrier-Katalysator enthaltend eine Eisenverbindung, eine Kaliumverbindung und eine Cerverbindung. Außerdem offenbart keines der Dokumente eine positive Wirkung von Mangan und/oder Titan auf die katalytische Leistung bei einem geringem molaren Dampf/Kohlenwasserstoff-Verhältnis von kleiner 7,35 (mol/mol).

Es besteht ein Bedarf an weiter verbesserten Dehydrier-Katalysatoren für die Dehydrierung von Kohlenwasserstoffen, die höhere Stabilität bei verbesserter oder gleichbleibender Katalysatoraktivität und somit höhere Standzeiten aufweisen. Eine Aufgabe der vorliegenden Erfindung besteht darin, einen verbesserten Dehydrier-Katalysator auf der Basis von Eisenoxid bereitzustellen, der sich insbesondere durch eine verbesserte Stabilität und/oder durch eine verbesserte Katalysatoraktivität auszeichnet.

Eine weitere Aufgabe der vorliegenden Erfindung besteht darin, verbesserte Dehydrier-Katalysatoren bereitzustellen, welche in Dehydrier-Verfahren mit geringen Dampf/Kohlenwasserstoff-Verhältnis (D/KW-Verhältnis), d.h. insbesondere bei molaren D/KW-Verhältnisses von kleiner oder gleich 7,35, eine bessere Stabilität und/oder Aktivität aufweisen als die Katalysator-Zusammensetzungen aus dem Stand der Technik.

Ebenso sollen eine ausreichende mechanische Stabilität des Katalysators und Beständigkeit gegen Siedewasser gegeben sein. Zudem sollte die Herstellung des Dehydrier-Katalysators einfach und kostengünstig durchzuführen sein, insbesondere sollten keine aufwändigen Verfahrensschritte, wie beispielsweise ein Sol-Gel-Verfahren, und/oder hohen Kalzinierungstemperaturen bei der Herstellung des Katalysators erforderlich sein.

Es wurde nun überraschenderweise gefunden, dass sich Dehydrier-Katalysatoren enthaltend 0,7 bis 10 Gew.-% einer Manganverbindung in Dehydrier-Verfahren mit geringem D/KW-Verhältnis (molares D/KW-Verhältnis von kleiner oder gleich 7,35, insbesondere kleiner oder gleich 6) vorteilhaft, d.h. mit ausreichender Standzeit und hohen Ausbeuten, einsetzen lassen.

Es wurde zudem gefunden, dass durch Zugabe von geringen Mengen von Titan in Dehydrier-Katalysatoren, welche Eisenoxid, eine Kaliumverbindung, eine Cerverbindung und einen Manganverbindung enthalten, die promotierende Wirkung von Mangan insbesondere verstärkt werden kann. In diesem Zusammenhang konnte eine optimale Menge an Titan ermittelt werden. Es wurde gefunden, dass die promotierende Wirkung des Mangans bei einer Erhöhung der Titanmenge in eine inhibierende Wirkung umschlagen kann.

Gegenstand der Erfindung ist ein Dehydrier-Katalysator enthaltend mindestens eine Eisenverbindung, mindestens eine Kaliumverbindung, mindestens eine Cerverbindung, 0,1 bis 10 Gew.-% mindestens einer Calciumverbindung, gerechnet als CaO, 0,7 bis 10 Gew.-%, bevorzugt 0,7 bis 5 Gew.-%, besonders bevorzugt 0,7 bis 3 Gew.-%, insbesondere bevorzugt 0,7 bis 2 Gew.-%, insbesondere bevorzugt 1 bis 2 Gew.-%, mindestens einer Manganverbindung, gerechnet als $MnO_2$ und 30 bis 150 ppm, bevorzugt 50 bis 120 ppm, insbesondere bevorzugt 60 bis 100 ppm, ganz besonders bevorzugt 60 bis 80 ppm, mindestens einer Titanverbindung, gerechnet als $TiO_2$.

Soweit nicht anders angegeben, beziehen sich alle folgenden Angaben in Gew.-% auf den gesamten Dehydrier-Katalysator und sind jeweils gerechnet auf das Metalloxid in der höchsten Oxidationsstufe. Im Sinne der vorliegenden Erfindung ist ppm als Milligramm pro Kilogramm (mg/kg) zu verstehen.

Die erfindungsgemäßen Katalysatoren zeichnen sich durch eine verbesserte Aktivität und Stabilität aus im Vergleich zu den im Stand der Technik beschriebenen Katalysatoren. Insbesondere zeigen die erfindungsgemäßen Katalysatoren verbesserte Eigenschaften bei Dehydrier-Verfahren mit einem niedrigen Dampf/Kohlenwasserstoff-Verhältnis (D/KW-Verhältnis), d.h. bei molaren D/KW-Verhältnissen von kleiner oder gleich 7,35. Diese verbesserte Aktivität bei niedrigem D/KW-Verhältnis zeigt sich beispielsweise auch in einem geringeren Abfall der Aktivität im Vergleich zu bekannten Katalysatoren, wenn man von mittleren zu niedrigen D/KW-Verhältnissen übergeht.

Dehydrier-Katalysator enthaltend mindestens eine Eisenverbindung, mindestens eine Kaliumverbindung, mindestens eine Cerverbindung und mindestens eine Manganverbindung und optional weitere Metallverbindungen ist im Sinne der vorliegenden Erfindung so zu verstehen, dass die entsprechenden Metalle in den gegebenenfalls angegebenen Mengen im Katalysator bestimmt werden können. Im Katalysator können typischerweise Mischphasen (z.B. Oxid-Mischphasen) und/oder isolierte Phasen der im Folgenden beschriebenen Metallverbindungen vorliegen. Es ist auch möglich, dass eine oder mehrere der im folgenden beschriebenen Komponente(n) teilweise oder vollständig in einem anderen bei der Katalysator-Herstellung verwendeten Rohstoff enthalten ist/sind.

Erfindungsgemäß enthält der Dehydrier-Katalysator mindestens eine Eisenverbindung bzw. wird bei der Herstellung des Dehydrier-Katalysators mindestens eine Eisenverbindung eingesetzt. Die mindestens eine Eisenverbindung ist bevorzugt ein Eisenoxid, insbesondere $Fe_2O_3$. Bevorzugt ist die mindestens eine Eisenverbindung ausgewählt aus natürlichen oder synthetischen Eisenoxiden und/oder Eisenoxidhydroxiden. Insbesondere ist die mindestens eine Eisenverbindung ausgewählt aus der Gruppe bestehend aus $\alpha$-$Fe_2O_3$ (Hämatit), $\gamma$-$Fe_2O_3$, Eisenoxidhydroxid (z.B. $\alpha$-FeOOH, Goethit) und $Fe_3O_4$ (Magnetit). Als synthetische Eisenoxide können beispielsweise Eisenoxide eingesetzt werden, die durch thermische Zersetzung von Eisensalzlösungen hergestellt wurden.

Insbesondere kann die mindestens eine Eisenverbindung und die mindestens eine Kaliumverbindung in Form einer Kaliumferrit-Phase $K_xFe_yO_z$ (mit x = 1-2, y = 1-22 und z = 2-34) vorliegen.

Bevorzugt wird als Eisenverbindung $\alpha$-$Fe_2O_3$ (Hämatit) eingesetzt. Bevorzugt ist auch der Einsatz von $\alpha$-$Fe_2O_3$ (Hämatit) in Kombination mit Goethit (FeOOH) und/oder Magnetit ($Fe_3O_4$) als Eisenverbindung. Der Anteil an Goethit (FeOOH) und/oder Magnetit ($Fe_3O_4$) beträgt dann typischerweise 0 bis 30 Gew.-% (bezogen auf die Gesamtmenge an Eisenverbindung).

Die spezifische Oberfläche der Eisenverbindung (z.B. mittels BET-Methode bestimmt) liegt typischerweise im Bereich von 1 bis 50 $m^2$/g, bevorzugt von 1 bis 20 $m^2$/g.

Die mindestens eine Eisenverbindung ist typischerweise in einer Menge im Bereich von 50 bis 90 Gew.-%, bevorzugt 60 bis 80 Gew.-%, besonders bevorzugt von 65 bis 75 Gew.-%, gerechnet als $Fe_2O_3$, im Dehydrier-Katalysator enthalten (bezogen auf das Gesamtgewicht des Dehydrier-Katalysators).

Erfindungsgemäß enthält der Katalysator mindestens eine Kaliumverbindung bzw. wird bei der Herstellung des Dehydrier-Katalysators mindestens eine Kaliumverbindung eingesetzt. Die mindestens eine Kaliumverbindung ist bevorzugt ausgewählt aus Kaliumoxid, Kaliumcarbonat, Kaliumhydroxid, Kaliumhydrogencarbonat, Kaliumoxalat und Kaliumferrit, insbesondere ausgewählt aus Kaliumoxid, Kaliumcarbonat, Kaliumhydroxid und Kaliumhydrogencarbonat. Die mindestens eine Kaliumverbindung ist insbesondere ein Kaliumoxid ($K_2O$) oder ein Mischoxid. Es kann auch eine andere in der Wärme zersetzbaren Kaliumverbindung verwendet werden. Die mindestens eine Kaliumverbindung kann in dem Katalysator typischerweise als Oxid-Mischphase mit den im Katalysator vorhandenen Metallen vorliegen.

Die mindestens eine Kaliumverbindung ist typischerweise in einer Menge im Bereich von 1 bis 30 Gew.-%,

4

bevorzugt von 5 bis 25 Gew.-%, insbesondere bevorzugt von 10 bis 15 Gew.-%, im Dehydrier-Katalysator enthalten (bezogen auf das Gesamtgewicht des Dehydrier-Katalysators und gerechnet als $K_2O$).

Erfindungsgemäß enthält der Dehydrier-Katalysator mindestens eine Cerverbindung bzw. wird bei der Herstellung des Dehydrier-Katalysators mindestens eine Cerverbindung eingesetzt. Die mindestens eine Cerverbindung ist bevorzugt ausgewählt aus Ceroxiden, Cerhydroxiden, Cercarbonaten, wasserhaltigen Cercarbonat und Ceroxalaten. Bevorzugt können Mischungen der genannten Cerverbindungen eingesetzt werden. Bevorzugt ist die mindestens eine Cerverbindung ausgewählt aus Cer(IV)-oxid ($CeO_2$), Cer(III)-oxalat und Cer(III)-carbonat, bevorzugt aus Cer(IV)-oxid ($CeO_2$) und Cer(III)-carbonat. Typischerweise wird die mindestens eine Cerverbindung bei der Herstellung des Katalysators in Cerdioxid umgewandelt.

Bevorzugt enthält der Dehydrier-Katalysator 2 bis 20 Gew.-%, bevorzugt 5 bis 15 Gew.-%, insbesondere bevorzugt 5 bis 10 Gew.-%, mindestens einer Cerverbindung, gerechnet als $CeO_2$.

Erfindungsgemäß enthält der Katalysator 0,7 bis 10 Gew.-% mindestens einer Manganverbindung, gerechnet als $MnO_2$; bzw. es wird bei der Herstellung des Dehydrier-Katalysators mindestens eine Manganverbindung in der angegebenen Menge eingesetzt. Bevorzugt ist die mindestens eine Manganverbindung ausgewählt aus Manganoxiden (z. B. $MnO$, $Mn_2O_3$, $MnO_2$, $Mn_3O_4$), Mangancarbonaten und Permanganaten. Besonders bevorzugt ist die mindestens eine Manganverbindung ausgewählt aus der Gruppe bestehend aus $MnO$, $Mn_2O_3$, $MnO_2$ (z.B. Braunstein), $Mn_3O_4$ und $Mn_2O_7$. Insbesondere ist die mindestens eine Manganverbindung ein Manganoxid, insbesondere $MnO_2$.

Die mindestens eine Manganverbindung wird bei der Herstellung des Katalysators bevorzugt als Mangandioxid ($MnO_2$) zugegeben. Es ist aber auch möglich andere Oxide des Mangans oder auch andere Manganverbindungen einzusetzen, welche sich bei Wärme zersetzen. Es ist weiterhin möglich, dass die mindestens eine Manganverbindung bereits teilweise oder vollständig in einem anderen bei der Katalysatorherstellung verwendeten Rohstoff enthalten ist, z.B. im Eisenoxid.

Bevorzugt liegt das im Katalysator enthaltene Mangan nicht in Form eines Mangan-Eisen-Mischoxides vor. Bevorzugt liegen mindestens 80 Gew.-%, bevorzugt mindestens 90 Gew.-% (bezogen auf Gesamtmenge an Mangan) des im Katalysator enthaltenen Mangans nicht in Form eines Mangan-Eisen-Mischoxides vor. Bevorzugt bildet das Mangan im vorliegenden Katalysator teilweise oder nahezu vollständig eine eigenständige Manganoxidphase. Es ist auch möglich, dass das im Katalysator enthaltene Mangan teilweise oder nahezu vollständig in Form einer Mangan-Kalium-Mischoxidphase vorliegt.

Erfindungsgemäß enthält der Dehydrier-Katalysator 0,7 bis 10 Gew.-%, bevorzugt 0,7 bis 5 Gew.-%, besonders bevorzugt 0,7 bis 3 Gew.-%, insbesondere bevorzugt 0,7 bis 2 Gew.-%, insbesondere bevorzugt 1 bis 2 Gew.-% mindestens einer Manganverbindung, gerechnet als $MnO_2$ (bezogen auf den gesamten Katalysator).

Erfindungsgemäß enthält der Dehydrier-Katalysator 30 bis 150 ppm, bevorzugt 50 bis 120 ppm, insbesondere bevorzugt 60 bis 100 ppm, ganz besonders bevorzugt 60 bis 80 ppm, mindestens einer Titanverbindung, gerechnet als $TiO_2$, bzw. es wird bei der Herstellung des Dehydrier-Katalysators mindestens eine Titanverbindung eingesetzt.

Im Sinne der vorliegenden Erfindung ist ppm als Milligramm pro Kilogramm (mg/kg) zu verstehen
Die mindestens eine Titanverbindung kann insbesondere ausgewählt sein aus Titanoxiden, Titanalkoholaten und Titancarboxylaten. Bevorzugt ist die mindestens eine Titanverbindung Titandioxid ($TiO_2$). Die mindestens eine Titanverbindung wird bei der Herstellung des Katalysators bevorzugt als Titandioxid ($TiO_2$) zugegeben. Es ist aber auch möglich andere Titanverbindungen einzusetzen. Es ist weiterhin möglich, dass die mindestens eine Titanverbindung bereits teilweise oder vollständig in einem anderen bei der Katalysatorherstellung verwendeten Rohstoff enthalten ist, z.B. im Eisenoxid.

Es wurde gefunden, dass sich insbesondere durch die Kombination der mindestens einen Manganverbindung und der mindestens einer Titanverbindung ein Katalysator mit besonders vorteilhaften Eigenschaften herstellen lässt, wobei insbesondere in Bezug auf verbesserte Aktivität und Ausbeute ein synergistischer Effekt zu beobachten ist. Besonders vorteilhaft erwies sich die Zugabe von 30 bis 150 ppm mindestens einer Titanverbindung, gerechnet als $TiO_2$. Es wurde zudem gefunden, dass die Zugabe von Titan ohne die Zugabe von Mangan eine wesentlich geringere Verbesserung der Katalysator-Aktivität bewirkt.

Die vorliegende Erfindung betrifft daher einen Dehydrier-Katalysator enthaltend 0,7 bis 10 Gew.-%, bevorzugt 0,7 bis 5 Gew.-%, besonders bevorzugt 0,7 bis 3 Gew.-%, insbesondere bevorzugt 0,7 bis 2 Gew.-%, insbesondere bevorzugt 1 bis 2 Gew.-% mindestens einer Manganverbindung, gerechnet als $MnO_2$, und 30 bis 150 ppm, bevorzugt 50 bis 120 ppm, insbesondere bevorzugt 60 bis 100 ppm, ganz besonders bevorzugt 60 bis 80 ppm, mindestens einer Titanverbindung, gerechnet als $TiO_2$ .

In einer besonders bevorzugten Ausführungsform betrifft die vorliegende Erfindung einen Dehydrier-Katalysator enthaltend 0,7 bis 3 Gew.-% mindestens einer Manganverbindung, gerechnet als $MnO_2$, und 50 bis 120 ppm mindestens einer Titanverbindung, gerechnet als $TiO_2$.

In einer besonders bevorzugten Ausführungsform betrifft die vorliegende Erfindung einen Dehydrier-Katalysator enthaltend 0,7 bis 2 Gew.-% mindestens einer Manganverbindung, gerechnet als $MnO_2$, und 60 bis 100 ppm mindestens einer Titanverbindung, gerechnet als $TiO_2$.

In einer bevorzugten Ausführungsform bezieht sich die vorliegende Erfindung auf einen Dehydrier-Katalysator wie oben beschrieben enthaltend:

50 bis 90 Gew.-% mindestens einer Eisenverbindung, gerechnet als $Fe_2O_3$;

1 bis 30 Gew.-% mindestens einer Kaliumverbindung, gerechnet als $K_2O$;

0,7 bis 10 Gew.-%, bevorzugt 0,7 bis 5 Gew.-%, besonders bevorzugt 0,7 bis 3 Gew.-%, insbesondere bevorzugt 0,7 bis 2 Gew.-%, insbesondere bevorzugt 1 bis 2 Gew.-% mindestens einer Manganverbindung gerechnet als $MnO_2$;

10 bis 200 ppm, bevorzugt 30 bis 150 ppm, besonders bevorzugt 50 bis 120 ppm, insbesondere bevorzugt 60 bis 100 ppm, ganz besonders bevorzugt 60 bis 80 ppm, mindestens einer Titanverbindung, gerechnet als $TiO_2$;

2 bis 20 Gew.-% mindestens einer Cerverbindung, gerechnet als $CeO_2$; und

optional 0 bis 30 Gew.-% mindestens einer weiteren Komponente.

In einer bevorzugten Ausführungsform addieren sich die oben genannten Komponenten zu 100 Gew.-%.

Die weiteren Komponenten können in Mengen von 0 bis 30 Gew.-%, bevorzugt von 0 bis 20 Gew.-%, bevorzugt von 0,001 bis 10 Gew.-%, insbesondere bevorzugt von 0,1 bis 5 Gew.-%, insbesondere bevorzugt von 0,5 bis 5 Gew.-% enthalten sein (bzw. zugesetzt werden).

Bevorzugt kann der Dehydrier-Katalysator als weitere Komponente mindestens eine Verbindung ausgewählt aus der Gruppe bestehend aus Molybdän (Mo), Wolfram (W) und Vanadium (V) enthalten; bzw. es wird bei der Herstellung des Dehydrier-Katalysators mindestens eine solche Verbindung eingesetzt. Die weitere Komponente kann insbesondere ausgewählt werden aus Sauerstoffverbindungen (beispielsweise Oxiden, Oxidhydraten, Oxoverbindungen) von Molybdän (Mo), Wolfram (W) und Vanadium (V). Insbesondere handelt es sich bei der mindestens eine Verbindung ausgewählt aus der Gruppe bestehend aus Molybdän (Mo), Wolfram (W) und Vanadium (V) um eine Verbindung, die unter Hitzeeinwirkung bei der Herstellung des Dehydrier-Katalysators zerfällt.

Bevorzugt wird als mindestens eine weitere Komponente mindestens eine Molybdänverbindung eingesetzt ausgewählt aus Molybdänoxiden und Molybdaten (z.B. Ammoniummolybdat, Kaliummolybdat). Bevorzugt handelt es sich bei der mindestens einen Molybdänverbindung um Ammoniumheptamolybdat.

Bevorzugt enthält der Dehydrier-Katalysator als weitere Komponente 0,1 bis 10 Gew.-%, bevorzugt 1 bis 5 Gew.-%, mindestens einer Verbindung ausgewählt aus der Gruppe bestehend aus Molybdän, Wolfram und Vanadium, gerechnet als Oxid in der jeweils höchsten Oxidationsstufe.

Insbesondere bevorzugt enthält der Dehydrier-Katalysator als weitere Komponente 0,1 bis 10 Gew.-%, bevorzugt 1 bis 5 Gew.-% mindestens einer Molybdänverbindung, gerechnet als $MoO_3$.

Weiterhin kann der Dehydrier-Katalysator 0 bis 10 Gew.-%, besonders bevorzugt 1 bis 5 Gew.-%, mindestens einer Vanadiumverbindung, gerechnet als $V_2O_5$, enthalten.

Bevorzugt enthält der Dehydrier-Katalysator als weitere Komponenten mindestens eine Erdalkalimetallverbindung, bzw. es wird bei der Herstellung des Dehydrier-Katalysators mindestens eine Erdalkalimetallverbindung eingesetzt. Insbesondere kann der Dehydrier-Katalysator als weitere Komponente 0,1 bis 10 Gew.-%, bevorzugt 1 bis 5 Gew.-% mindestens einer Erdalkalimetallverbindung, gerechnet als Oxid, enthalten.

In einer bevorzugten Ausführungsform enthält der Dehydrier-Katalysator mindestens eine Magnesiumverbindung als weitere Komponente. Bevorzugt enthält der Dehydrier-Katalysator als weitere Komponente 0,1 bis 10 Gew.-%, bevorzugt 1 bis 5 Gew.-% mindestens einer Magnesiumverbindung, gerechnet als MgO. Insbesondere ist die mindestens eine Magnesiumverbindung ausgewählt aus Magnesiumoxid, Magnesiumcarbonat (z.B. Magnesit) und Magnesiumhydroxid. Bevorzugt handelt es sich bei der mindestens einen Magnesiumverbindung um Magnesiumoxid (MgO) und/oder Magnesiumcarbonat ($MgCO_3$) (z.B. Magnesit). Bevorzugt wird bei der Herstellung des Katalysators als weitere Komponente Magnesiumoxid (MgO) und/oder Magnesiumcarbonat ($MgCO_3$) (z.B. Magnesit) eingesetzt.

Erfindungsgemäß enthält der Dehydrier-Katalysator 0,1 bis 10 Gew.-%, bevorzugt 1 bis 5 Gew.-% mindestens einer Calciumverbindung, gerechnet als CaO. Insbesondere ist die mindestens eine Calciumverbindung ausgewählt aus Calciumoxid, Calciumcarbonat und Calciumhydroxid. Bevorzugt handelt es sich bei der mindestens einen Calciumverbindung um Calciumoxid (CaO). Bevorzugt wird bei der Herstellung des Katalysators als weitere Komponente Calciumoxid (CaO) und/oder Calciumhydroxid ($Ca(OH)_2$) eingesetzt.

In einer bevorzugten Ausführungsform enthält der Dehydrier-Katalysator mindestens eine Magnesiumverbindung und mindestens eine Calciumverbindung. Insbesondere enthält der Dehydrier-Katalysator 0,1 bis 10 Gew.-%, bevorzugt 1 bis 5 Gew.-% mindestens einer Magnesiumverbindung, gerechnet als MgO, und 0,1 bis 10 Gew.-%, bevorzugt 1 bis 5 Gew.-% mindestens einer Calciumverbindung, gerechnet als CaO.

In einer bevorzugten Ausführungsform enthält der oben beschriebenen Dehydrier-Katalysator als weitere Komponenten:

0,1 bis 10 Gew.-%, bevorzugt 1 bis 5 Gew.-%, mindestens einer Erdalkalimetallverbindungen, gerechnet als Oxid, und

0,1 bis 10 Gew.-%, bevorzugt 1 bis 5 Gew.-%, mindestens einer Verbindung ausgewählt aus der Gruppe bestehend aus Molybdän (Mo), Wolfram (W) und Vanadium (V) (jeweils gerechnet als das Oxid in der höchsten Oxidationsstufe).

In einer weiteren bevorzugten Ausführungsform enthält der oben beschriebenen Dehydrier-Katalysator als weitere Komponenten:

0,1 bis 10 Gew.-%, bevorzugt 1 bis 5 Gew.-% mindestens einer Magnesiumverbindung gerechnet als MgO;

0,1 bis 10 Gew.-%, bevorzugt 1 bis 5 Gew.-%, mindestens einer Calciumverbindung, gerechnet als CaO;

0,1 bis 10 Gew.-%, bevorzugt 1 bis 5 Gew.-%, mindestens einer Molybdänverbindung, gerechnet als $MoO_3$.

Weiterhin kann der Dehydrier-Katalysator als mindestens eine weitere Komponente (als Promotor oder Dotierstoff) einen oder mehrere der üblichen Verbindungen zur Steigerung der Aktivität und/oder Selektivität enthalten, beispielsweise Verbindungen ausgewählt von Cr, Co, Ni, Cu, Zn, Al, Ga, Ge, Zr, Nb, Ru, Rh, Pd, Ag, Cd, In, Sn, Sb, La, Hf, Ta, Re, Ir, Pt, Au, Pb und Bi. Die genannten üblichen Promotoren können typischerweise in Mengen von 0 bis 10 Gew.-%, vorzugsweise von 0,001 bis 5 Gew.-%, vorzugsweise von 0,01 bis 2 Gew.-% enthalten sein.

In einer Ausführungsform enthält der oben beschriebene Dehydrier-Katalysator als weitere Komponente mindestens eine weitere Seltenerdenmetallverbindung neben Cer, insbesondere ausgewählt aus der Gruppe bestehend aus Lanthan (La), Praseodym (Pr) und Neodym (Nd). Bevorzugt enthält der Dehydrier-Katalysator 1 bis 1.000 ppm, bevorzugt 10 bis 500 ppm, besonders bevorzugt 20 bis 300 ppm, mindestens einer weiteren Seltenerdenmetallverbindung, neben Cer, gerechnet als Oxid in der jeweils höchsten Oxidationsstufe. Insbesondere enthält der Katalysator 1 bis 1.000 ppm, bevorzugt 10 bis 500 ppm, besonders bevorzugt 20 bis 300 ppm, mindestens einer Seltenerdenmetallverbindung ausgewählt aus der Gruppe bestehend aus Lanthan, Praseodym und Neodym. Bevorzugt kann der Dehydrier-Katalysator als weitere Komponente 1 bis 1.000 ppm, bevorzugt 3 bis 500, besonders bevorzugt 10 bis 100 ppm mindestens einer Lanthanverbindung, gerechnet als $La_2O_3$, enthalten. Bevorzugt kann der Dehydrier-Katalysator als weitere Komponente 1 bis 1.000 ppm, bevorzugt 3 bis 500, besonders bevorzugt 10 bis 100 ppm mindestens einer Praseodymverbindung, gerechnet als $PrO_2$, enthalten. Bevorzugt kann der Dehydrier-Katalysator als weitere Komponente 1 bis 1.000 ppm, bevorzugt 3 bis 500, besonders bevorzugt 10 bis 100 ppm mindestens einer Neodymverbindung, gerechnet als $Nd_2O_3$, enthalten.

Der oben beschriebene Dehydrier-Katalysator kann vorzugsweise als weitere Komponente mindestens eine Verbindung von Metallen der 8. bis 12. Nebengruppe des Periodensystems der Elemente enthalten. Bevorzugt enthält der oben beschriebene Dehydrier-Katalysator als weitere Komponente mindestens eine Verbindung von Metallen ausgewählt aus der Gruppe bestehend aus Ruthenium (Ru), Osmium (Os), Kobalt (Co), Nickel (Ni), Palladium (Pd), Platin (Pt), Kupfer (Cu), Silber (Ag), Gold (Au) und Zink (Zn); bevorzugt ausgewählt aus der Gruppe bestehend aus Kobalt (Co), Nickel (Ni), Palladium (Pd), Kupfer (Cu), und Zink (Zn); besonders bevorzugt ausgewählt aus der Gruppe bestehend aus Nickel (Ni), Kupfer (Cu), und Zink (Zn). Der oben beschriebene Dehydrier-Katalysator kann als weitere Komponente insbesondere 1 bis 1.000 ppm, bevorzugt 50 bis 500 ppm, besonders bevorzugt 50 bis 200 ppm, mindestens einer Verbindung von Metallen der 8. bis 12. Nebengruppe des Periodensystems der Elemente, gerechnet jeweils als Oxid in der höchsten Oxidationsstufe, enthalten. In einer bevorzugten Ausführungsform enthält der oben beschriebene Dehydrier-Katalysator 1 bis 1.000 ppm, bevorzugt 50 bis 500 ppm, besonders bevorzugt 50 bis 200 ppm, mindestens einer Verbindung von Metallen ausgewählt aus der Gruppe bestehend aus Nickel (Ni), Kupfer (Cu), und Zink (Zn), gerechnet jeweils als das Oxid in der höchsten Oxidationsstufe. Bevorzugt kann der Dehydrier-Katalysator als weitere Komponente 1 bis 1.000 ppm, bevorzugt 30 bis 500 ppm, besonders bevorzugt 30 bis 200 ppm, mindestens einer Nickelverbindung, gerechnet als NiO, enthalten. Bevorzugt kann der Dehydrier-Katalysator als weitere Komponente 1 bis 1000 ppm, bevorzugt 10 bis 200, besonders bevorzugt 30 bis 100 ppm mindestens einer Kupferverbindung, gerechnet als CuO, enthalten. Bevorzugt kann der Dehydrier-Katalysator als weitere Komponente 1 bis 1.000 ppm, bevorzugt 1 bis 500 ppm, besonders bevorzugt 10 bis 100 ppm, mindestens einer Zinkverbindung, gerechnet als ZnO, enthalten.

Darüberhinaus kann der Dehydrier-Katalysator als weitere Komponente mindestens eine Verbindung von Elementen der 4. Hauptgruppe des Periodensystems der Elemente enthalten. Bevorzugt enthält der oben beschriebene Dehydrier-Katalysator als weitere Komponente mindestens eine Verbindung ausgewählt aus der Gruppe bestehend aus Silicium (Si)-, Germanium (Ge)-, Zinn (Sn)-, und Blei (Pb)-Verbindungen, bevorzugt mindestens eine Siliciumverbindung. Insbesondere enthält der Dehydrier-Katalysator 1 bis 1.000 ppm, bevorzugt 5 bis 500 ppm, besonders bevorzugt 10 bis 100 ppm, mindestens einer Verbindung von ausgewählt aus der Gruppe bestehend aus Silicium (Si)-, Germanium (Ge)-, Zinn (Sn)-, und Blei (Pb)-Verbindungen, gerechnet als Oxid in der jeweils höchsten Oxidationsstufe. In einer Ausführungsform enthält der beschriebene Dehydrier-Katalysator 1 bis 1000 ppm, bevorzugt 5 bis 500 ppm, besonders bevorzugt 10 bis 100 ppm, mindestens einer Siliciumverbindung, gerechnet als $SiO_2$.

Typischerweise kann der oben beschriebene Dehydrier-Katalysator als Nichtmetall, neben Sauerstoff, mindestens ein Nicht-Metall ausgewählt aus Nichtmetallen der 5. bis 7. Hauptgruppe des Periodensystems der Elemente enthalten, insbesondere ausgewählt aus der Gruppe bestehend aus Stickstoff, Phosphor, Schwefel und Chlor.

In einer bevorzugten Ausführungsform enthält der Dehydrier-Katalysator:

50 bis 90 Gew.-%, besonders bevorzugt 60 bis 80 Gew.-%, mindestens einer Eisenverbindung, gerechnet als $Fe_2O_3$;

1 bis 30 Gew.-%, besonders bevorzugt 5 bis 25 Gew.-%, mindestens einer Kaliumverbindung, gerechnet als $K_2O$;

0,7 bis 10 Gew.-%, bevorzugt 0,7 bis 5 Gew.-%, besonders bevorzugt 0,7 bis 3 Gew.-%, insbesondere bevorzugt 0,7 bis 2 Gew.-%, besonders bevorzugt 1 bis 2 Gew.-%, mindestens einer Manganverbindung, gerechnet als $MnO_2$;

10 bis 200 ppm, bevorzugt 30 bis 150 ppm, besonders bevorzugt 50 bis 120 ppm, insbesondere bevorzugt 60 bis 100, ganz besonders bevorzugt 60 bis 80 ppm mindestens einer Titanverbindung, gerechnet als $TiO_2$;

2 bis 20 Gew.-% besonders bevorzugt 5 bis 15 Gew.-%, mindestens einer Cerverbindung, gerechnet als $CeO_2$;

0,1 bis 10 Gew.-%, besonders bevorzugt 1 bis 5 Gew.-%, mindestens einer Magnesiumverbindung, gerechnet als MgO;

0,1 bis 10 Gew.%, besonders bevorzugt 1 bis 5 Gew.-%, mindestens einer Calciumverbindung, gerechnet als CaO;

0,1 bis 10 Gew.-%, besonders bevorzugt 1 bis 5 Gew.-%, mindestens einer Molybdänverbindung, gerechnet als $MoO_3$;

0 bis 10 Gew.-%, besonders bevorzugt 1 bis 5 Gew.-%, mindestens einer Vanadiumverbindung, gerechnet als $V_2O_5$ und

0 bis 10 Gew.-% mindestens einer weiteren Komponente.

In einer bevorzugten Ausführungsform ergeben die oben genannten Komponenten 100 Gew.-%.

In einer bevorzugten Ausführungsform enthält der Dehydrier-Katalysator:

50 bis 90 Gew.-%, besonders bevorzugt 60 bis 80 Gew.-%, mindestens einer Eisenverbindung, gerechnet als $Fe_2O_3$;

1 bis 30 Gew.-%, besonders bevorzugt 5 bis 25 Gew.-%, mindestens einer Kaliumverbindung, gerechnet als $K_2O$;

0,7 bis 10 Gew.-%, bevorzugt 0,7 bis 5 Gew.-%, besonders bevorzugt 0,7 bis 3 Gew.-%, insbesondere bevorzugt 0,7 bis 2 Gew.-%, besonders bevorzugt 1 bis 2 Gew.-%, mindestens einer Manganverbindung, gerechnet als $MnO_2$;

10 bis 200 ppm, bevorzugt 30 bis 150 ppm, besonders bevorzugt 50 bis 120 ppm, insbesondere bevorzugt 60 bis 100, ganz besonders bevorzugt 60 bis 80 ppm mindestens einer Titanverbindung, gerechnet als $TiO_2$;

2 bis 20 Gew.-% besonders bevorzugt 5 bis 15 Gew.-%, mindestens einer Cerverbindung, gerechnet als $CeO_2$;

0,1 bis 10 Gew.-%, besonders bevorzugt 1 bis 5 Gew.-%, mindestens einer Magnesiumverbindung, gerechnet als MgO;

0,1 bis 10 Gew.-%, besonders bevorzugt 1 bis 5 Gew.-%, mindestens einer Calciumverbindung, gerechnet als CaO;

0,1 bis 10 Gew.-%, besonders bevorzugt 1 bis 5 Gew.-%, mindestens einer Molybdänverbindung, gerechnet als $MoO_3$;

0 bis 10 Gew.-%, besonders bevorzugt 1 bis 5 Gew.-%, mindestens einer Vanadiumverbindung, gerechnet als $V_2O_5$ und

1 bis 10.000 ppm, bevorzugt 10 bis 5.000 ppm, insbesondere bevorzugt 10 bis 3.000 ppm mindestens einer weiteren Komponente ausgewählt aus Lanthanverbindungen, Praseodymverbindungen, Neodymverbindungen, Nickelverbindungen, Kupferverbindungen, Zinkverbindungen und Silicium-verbindungen, gerechnet jeweils als Oxid in der höchsten Oxidationsstufe.

In einer bevorzugten Ausführungsform ergeben die oben genannten Komponenten 100 Gew.%.

Alle Angaben in Gew.-% beziehen sich - soweit nicht anders angegeben - auf den gesamten Dehydrier-Katalysator. Alle Angaben in Gew.-% wurden - soweit nicht anders angegeben - auf das Oxid des betreffenden Metalls jeweils in der höchsten Oxidationsstufe berechnet.

Insbesondere bezieht sich die vorliegende Erfindung auf einen oben beschriebenen Dehydrier-Katalysator zur katalytischen Dehydrierung von Kohlenwasserstoffen bei einem molaren Dampf/Kohlenwasserstoff-Gewichtsverhältnis im Bereich von 3 bis 7,35; bevorzugt im Bereich von 4 bis 7; insbesondere bevorzugt im Bereich von 5 bis 6.

Weiterhin bezieht sich die vorliegende Erfindung auf ein Verfahren zur Herstellung eines oben beschriebenen Dehydrier-Katalysators umfassend die folgenden Schritte:

i) Herstellen einer Katalysator-Vormischung durch Vermischen mindestens einer Eisenverbindung, mindestens einer Kaliumverbindung, mindestens einer Cerverbindung, 0,1 bis 10 Gew.-%, bezogen auf den fertigen Katalysator, mindestens einer Calciumverbindung gerechnet als CaO, 0,7 bis 10 Gew.-%, bezogen auf den fertigen Katalysator, mindestens einer Manganverbindung, gerechnet als $MnO_2$, 30 bis 150 ppm, bezogen auf den fertigen Katalysator, mindestens einer Titanverbindung, gerechnet als $TiO_2$, optional weiterer Metallverbindungen, optional weiterer Komponenten und optional mindestens eines Bindemittels mit einem Lösemittel;

ii) Herstellen von Katalysator-Formkörpern aus der in Schritt i) erhaltenen Katalysator-Vormischung;

iii) Trocknen der Katalysator-Formkörper und Kalzinieren der Katalysator-Formkörper.

Die grundlegende Vorgehensweise bei der Herstellung von Dehydrier-Katalysatoren ist dem Fachmann bekannt. Die Herstellung der oben beschriebenen Dehydrier-Katalysatoren kann beispielsweise wie in WO 99/49966 beschrieben erfolgen.

Für das Verfahren zur Herstellung eines Dehydrier-Katalysators werden bevorzugt die oben im Zusammenhang mit dem Dehydrier-Katalysator beschriebenen Metallverbindungverbindungen und weitere Komponenten eingesetzt. Insbesondere werden bei der Herstellung des Dehydrier-Katalysators die oben beschriebenen Eisenverbindungen, Kaliumverbindungen, Cerverbindungen, Calciumverbindungen, Manganverbindungen und Titanverbindungen eingesetzt. Optional können bei der Herstellung des Dehydrier-Katalysators insbesondere die oben beschriebenen Molybdänverbindungen, Vanadiumverbindungen, Magnesiumverbindungen und Calciumverbindungen eingesetzt werden. Optional können die oben beschriebenen weiteren Metallverbindungen bei der Herstellung eingesetzt werden, bevorzugt befindet sich eine oder mehrere der weiteren Metallverbindungen ganz oder teilweise in einem der eingesetzten Rohstoffe, insbesondere im Eisenoxid und/oder Cercarbonat.

Als Verbindungen und weitere Komponenten können Verbindungen, wie sie im fertigen Katalysator vorliegen, eingesetzt werden, oder Verbindungen, die sich während des Herstellungsprozess in Verbindungen umwandeln, wie sie im fertigen Katalysator vorliegen.

Insbesondere betrifft die Erfindung ein Verfahren zur Herstellung eines Dehydrier-Katalysators wie beschrieben, wobei als mindestens eine Eisenkomponente ein Eisen(III)-oxid ($Fe_2O_3$) eingesetzt wird. Insbesondere kann ein Eisenoxid mit der folgenden Zusammensetzung eingesetzt werden, wobei die Angaben die Menge des Elementes oder der Verbindungen bezogen auf die Gesamtmenge Eisenoxid angeben:

95 bis 99,99 Gew.-%, bevorzugt 98 bis 99,99 Gew.-% Eisen(III)-oxid ($Fe_2O_3$);
1 bis 10.000 ppm, bevorzugt 1 bis 5.000 ppm, besonders bevorzugt 1 bis 1.000 ppm Mangan (Mn);
0 bis 1.000 ppm, bevorzugt 1 bis 500 ppm, besonders bevorzugt 1 bis 100 ppm Natrium (Na);
0 bis 1.000 ppm, bevorzugt 1 bis 500 ppm, besonders bevorzugt 1 bis 50 ppm Calcium (Ca);
0 bis 1.000 ppm, bevorzugt 1 bis 500 ppm, besonders bevorzugt 1 bis 100 ppm Kupfer (Cu);
0 bis 1.000 ppm, bevorzugt 1 bis 500 ppm, besonders bevorzugt 1 bis 100 ppm Nickel (Ni);
0 bis 1.000 ppm, bevorzugt 1 bis 500 ppm, besonders bevorzugt 1 bis 100 ppm Zink (Zn);
0 bis 300 ppm, bevorzugt 1 bis 200 ppm, besonders bevorzugt 1 bis 100 ppm Titan (Ti);
0 bis 1.000 ppm, bevorzugt 1 bis 500 ppm, besonders bevorzugt 1 bis 100 ppm Chrom (Cr);

0 bis 1.000, ppm bevorzugt 1 bis 500 ppm, besonders bevorzugt 1 bis 100 ppm Silicium (Si);

0 bis 1.000 ppm, bevorzugt 1 bis 500 ppm, besonders bevorzugt 1 bis 100 ppm Chlor (Cl);

0 bis 10.000 ppm, bevorzugt 1 bis 5.000 ppm, besonders bevorzugt 1 bis 1.000 ppm Schwefel (S).

Insbesondere betrifft die Erfindung ein Verfahren zur Herstellung eines Dehydrier-Katalysators wie beschrieben, wobei als mindestens eine Kaliumverbindung ein Kaliumcarbonat ($K_2CO_3$) eingesetzt wird. Insbesondere kann ein Kaliumcarbonat mit der folgenden Zusammensetzung eingesetzt werden, wobei die Angaben die Menge des Elementes oder der Verbindungen bezogen auf die Gesamtmenge Kaliumcarbonat angeben:

80 bis 99 Gew.-%, bevorzugt 80 bis 85 Gew.-% Kaliumcarbonat;

0 bis 1 Gew.-%, bevorzugt 0,01 bis 1 Gew.-%, bevorzugt 0,01 bis 0,5 Gew.-% Natrium (Na);

0 bis 100 ppm, bevorzugt 1 bis 50, besonders bevorzugt 1 bis 20 ppm, Eisen (Fe).

0 bis 100 ppm, bevorzugt 1 bis 50 ppm Chlor (Cl);

Insbesondere betrifft die Erfindung ein Verfahren zur Herstellung eines Dehydrier-Katalysators wie beschrieben, wobei als mindestens eine Cerverbindung ein Cer(III)-carbonat ($CeCO_3$) eingesetzt wird. Insbesondere kann ein Cercarbonat mit der folgenden Zusammensetzung eingesetzt werden, wobei die Angaben die Menge des Elementes oder der Verbindungen bezogen auf die Gesamtmenge Cercarbonat angeben:

40 bis 85 Gew.-%, bevorzugt 45 bis 65 Gew.-% $CeO_2$;

1 bis 1.000 ppm, bevorzugt 100 bis 500 ppm, besonders bevorzugt 100 bis 300 ppm, Lanthan (La);

1 bis 1.000 ppm, bevorzugt 100 bis 500 ppm, besonders bevorzugt 200 bis 500 ppm, Praseodym (Pr);

1 bis 1.000 ppm, bevorzugt 1 bis 100 ppm, besonders bevorzugt 5 bis 50 ppm, Neodym (Nd);

0 bis 20 ppm, bevorzugt 1 bis 10 ppm, Titan (Ti);

0 bis 1.000 ppm, bevorzugt 1 bis 100 ppm, besonders bevorzugt 1 bis 50 ppm, Calcium (Ca);

0 bis 100 ppm, bevorzugt 1 bis 10 ppm Chlor;

0 bis 10.000 ppm, bevorzugt 1 bis 8.000 ppm Nitrat.

Insbesondere betrifft die Erfindung ein Verfahren zur Herstellung eines Dehydrier-Katalysators wie beschrieben, wobei als mindestens eine Manganverbindung Mangan(IV)-oxid ($MnO_2$) eingesetzt wird. Insbesondere kann ein Mangandioxid mit der folgenden Zusammensetzung eingesetzt werden, wobei die Angaben die Menge an Element oder Verbindung bezogen auf die Gesamtmenge Mangandioxid angeben:

95 bis 99,99 Gew.-%, bevorzugt 98 bis 99,99 Gew.-% $MnO_2$;

0 bis 0,5 Gew.-%, bevorzugt 0,01 bis 0,2 Gew.-% Eisen (Fe).

Insbesondere betrifft die Erfindung ein Verfahren zur Herstellung eines Dehydrier-Katalysators wie beschrieben, wobei als mindestens eine Titanverbindung Titandioxid ($TiO_2$) eingesetzt wird.

Insbesondere betrifft die Erfindung ein Verfahren zur Herstellung eines Dehydrier-Katalysators wie beschrieben, wobei als mindestens eine Molybdatverbindung ein Ammoniumheptamolydat eingesetzt wird. Insbesondere kann ein Ammoniumheptamolydat mit der folgenden Zusammensetzung eingesetzt werden, wobei die Angaben die Menge an Element oder Verbindung bezogen auf die Gesamtmenge Ammoniumheptamolydat angeben:

80 bis 85 Gew.-% $MoO_3$;

0 bis 1.000 ppm, bevorzugt 1 bis 500 ppm, besonders bevorzugt 1 bis 200 ppm, Kalium (K);

0 bis 1.000 ppm, bevorzugt 1 bis 200 ppm, besonders bevorzugt 1 bis 100 ppm, Natrium (Na).

Insbesondere betrifft die Erfindung ein Verfahren zur Herstellung eines Dehydrier-Katalysators wie beschrieben, wobei als mindestens eine Erdalkaliverbindung Magnesiumoxid (MgO) oder Calciumhydroxid ($Ca(OH)_2$) oder beides eingesetzt werden. Insbesondere kann ein Magnesiumoxid mit der folgenden Zusammensetzung eingesetzt werden:

92 bis 95 Gew.-% MgO;

0 bis 3 Gew.-%, bevorzugt 0,1 bis 2 Gew.-% Calcium (Ca);

0 bis 2 Gew.-%, bevorzugt 0,01 bis 0,5 Gew.-% Silicium (Si);

0 bis 2 Gew.-%, bevorzugt 0,1 bis 1 Gew.-% Eisen (Fe).

Insbesondere kann ein Calciumhydroxid mit der folgenden Zusammensetzung eingesetzt werden:

70 bis 90 Gew.-%, bevorzugt 70 bis 75 Gew.-% CaO;
0 bis 2 Gew.-%, bevorzugt 0,1 bis 1 Gew.-% Magnesium (Mg).

Die Angaben beziehen sich auf die Menge an Element oder Verbindung bezogen auf die Gesamtmenge des Rohstoffs.

Zur Herstellung der Katalysator-Vormischung werden in der Regel die Komponenten (typischerweise in Form von festen Pulvern) vermischt und dann mit einem Lösemittel, insbesondere Wasser, gegebenenfalls unter Zugabe eines Bindemittels vermischt. Das Vermischen erfolgt bevorzugt durch ein inniges Vermischen, z.B. durch Verkneten, in einem Rührkessel, Mixmuller, Mixer, Kneter oder Extruder, bevorzugt in einem Mixmuller, Kneter oder Mixer.

Aus der so erhaltenen Katalysator-Vormischung werden dann typischerweise Katalysator-Formkörper hergestellt (z.B. durch Extrusion oder Pressen), die anschließend getrocknet und optional kalziniert werden.

Die mindestens eine Eisenverbindung, die mindestens eine Kaliumverbindung, die mindestens eine Cerverbindung, die mindestens eine Manganverbindung und die mindestens eine Titanverbindung (typischerweise in Form von festen Pulvern) werden optional mit weiteren Metallverbindungen (insbesondere mindestens einer Erdalkalimetallverbindung, mindestens einer Molybdänverbindung) zunächst vermischt und dann mit dem Lösemittel und optional mindestens einem Bindemittel vermischt.

Als Lösemittel wird insbesondere Wasser oder eine Mischung aus polaren Lösemitteln (z.B. Alkoholen, Estern) und Wasser eingesetzt. Als Bindemittel (auch Plastifizierhilfsmittel) können beispielsweise Alginat, Stärke, Carboxymethylcellulose, Hydroxyethylcellulose und Polyvinylalkohol eingesetzt werden. Typischerweise werden die Bindemittel in Form einer Lösung in Wasser eingesetzt.

Die Herstellung von Katalysator-Formkörpern aus der Katalysator-Vormischung erfolgt typischerweise durch extrudieren oder pressen (Tablettenpressen). Beispiele für Katalysator-Formkörper sind Zylinder (Pellets), Ringe, Sternkörper und Wabenkörper. Bevorzugt erfolgt die Herstellung von Katalysator-Formkörpern aus der in Schritt i) erhaltenen Katalysator-Vormischung mittels Extrusion.

Nach der Formgebung werden die feuchten Formkörper typischerweise bei Temperaturen von 50 °C bis 500 °C, bevorzugt von 80 bis 350°C getrocknet. Die Trocknung kann beispielsweise im Trockenschank (z.B. auf Blechen), in einer Trocknungstrommel, oder auf Bandtrockner stattfinden.

Bevorzugt werden die Katalysator-Formkörpern in Schritt iii) bei Temperaturen im Bereich von 500 °C bis 1200 °C, bevorzugt von 750 bis 1.000 °C, bevorzugt von 800 bis 900 °C, kalziniert. Die Kalzinierung wird vorzugsweise in einem Drehrohrofen durchgeführt.

In einer bevorzugten Ausführungsform umfasst das oben beschriebene erfindungsgemäße Verfahren zur Herstellung eines Dehydrier-Katalysators die folgenden Schritte:

i) Herstellen einer Katalysator-Vormischung durch Vermischen mindestens einer Eisenverbindung, mindestens einer Kaliumverbindung, mindestens einer Cerverbindung, 0,1 bis 10 Gew.-%, bezogen auf den fertigen Katalysator, mindestens einer Calciumverbindung gerechnet als CaO, 0,7 bis 10 Gew.-%, bezogen auf den fertigen Katalysator, mindestens einer Manganverbindung, gerechnet als $MnO_2$, 30 bis 150 ppm, bezogen auf den fertigen Katalysator, mindestens einer Titanverbindung, gerechnet als $TiO_2$, optional weiterer Metallverbindungen, optional weiterer Komponenten und optional mindestens eines Bindemittels mit Wasser;

ii) Herstellen von Katalysator-Formkörpern aus der in Schritt i) erhaltenen Katalysator-Vormischung mittels Extrusion;

iii) Trocknen der Katalysator-Formkörper bei Temperaturen im Bereich von 50 °C bis 500 °C und Kalzinieren der Katalysator-Formkörpern im Bereich von 500 bis 1200 °C, bevorzugt von 750 bis 1.000 °C, bevorzugt von 800 bis 900 °C.

Bevorzugt betrifft die vorliegende Erfindung ein Verfahren zur Herstellung eines Dehydrier-Katalysators wie oben beschrieben, wobei in Schritt i) 0,7 bis 10 Gew.-% Mangandioxid ($MnO_2$) bevorzugt 0,7 bis 5 Gew.-%, besonders bevorzugt 0,7 bis 3 Gew.-%, insbesondere bevorzugt 0,7 bis 2 Gew.-%, insbesondere bevorzugt 1 bis 2 Gew.-% (bezogen auf den fertigen Katalysator) Mangandioxid ($MnO_2$) und 30 bis 150 ppm, bevorzugt 50 bis 120 ppm, insbesondere bevorzugt 60 bis 100 ppm, ganz besonders bevorzugt 60 bis 80 ppm (bezogen auf den fertigen Katalysator) Titandioxid ($TiO_2$), gerechnet als $TiO_2$, zugegeben werden.

In einem weiteren Aspekt betrifft die vorliegende Erfindung ein Verfahren zur katalytischen Dehydrierung eines Kohlenwasserstoffs, wobei ein Gemisch aus Wasserdampf und mindestens einem Kohlenwasserstoff mit einem oben beschriebenen Dehydrier-Katalysator in Kontakt gebracht wird. Bevorzugt handelt es sich bei dem Kohlenwasserstoff um Ethylbenzol.

Insbesondere betrifft die Erfindung ein Verfahren zur katalytischen Dehydrierung eines Kohlenwasserstoffs, wobei bei geringen Dampf/Kohlenwasserstoff-Verhältnissen gearbeitet wird. Bevorzugt betrifft die vorliegende Erfindung ein Verfahren zur katalytischen Dehydrierung eines Kohlenwasserstoffs, wobei ein Gemisch aus Wasserdampf und mindestens einem Kohlenwasserstoff mit einem molaren Dampf/Kohlenwasserstoff-Verhältnis im Bereich von 3 bis 7,35; bevorzugt im Bereich von 4 bis 7; insbesondere bevorzugt im Bereich von 5 bis 6 eingesetzt wird. Insbesondere betrifft die Erfindung ein Verfahren zur katalytischen Dehydrierung von Ethylbenzol zu Styrol, wobei ein Gemisch aus Wasserdampf und Ethylbenzol mit einem Dampf/Kohlenwasserstoff-Gewichtsverhältnis im Bereich von 0,5 bis 1,25; bevorzugt von 0,7 bis 1,2; insbesondere bevorzugt von 0,85 bis 1,1; besonders bevorzugt von 0,9 bis 1,0 eingesetzt wird.

In einem weiteren Aspekt beschreibt die vorliegende Erfindung ein Verfahren zur katalytischen Dehydrierung eines Kohlenwasserstoffs, wobei ein Gemisch aus Wasserdampf und mindestens einem Kohlenwasserstoff mit einem molaren Dampf/Kohlenwasserstoff-Verhältnis im Bereich von 3 bis 7,35; bevorzugt im Bereich von 4 bis 7; insbesondere bevorzugt im Bereich von 5 bis 6 mit einem Dehydrier-Katalysator enthaltend

mindestens eine Eisenverbindung, mindestens eine Kaliumverbindung, mindestens eine Cerverbindung und 0,7 bis 10 Gew.-% mindestens einer Manganverbindung, gerechnet als $MnO_2$,

in Kontakt gebracht wird. Für die Eisenverbindung, die Kaliumverbindung, die Cerverbindung und die Manganverbindung gelten insbesondere die oben beschriebenen Ausführungsformen.

Das beschriebene Verfahren unter Verwendung eines Dehydrier-Katalysator enthaltend mindestens eine Eisenverbindung, mindestens eine Kaliumverbindung, mindestens eine Cerverbindung und 0,7 bis 10 Gew.-% mindestens einer Manganverbindung zeichnet sich durch eine verbesserte Styrol-Ausbeute bei niedrigen D/KW-Verhältnissen im Vergleich bekannten Verfahren bzw. Dehydrier-Katalysatoren aus.

Bei den beschriebenen Verfahren kann es sich um die Dehydrierung von alkylaromatischen oder aliphatischen Kohlenwasserstoffen handeln, bevorzugt handelt es sich um die Dehydrierung von alkylaromatischen Kohlenwasserstoffen. Bei den erfindungsgemäßen Verfahren zur Dehydrierung eines Kohlenwasserstoffs kann es sich beispielsweise um die Dehydrierung von Ethylbenzol zu Styrol, von Isopropylbenzol zu alpha-Methylstyrol, von Buten zu Butadien oder von Isoamylen zu Isopren handeln. Bevorzugt handelt es sich bei dem Kohlenwasserstoff um Ethylbenzol.

Typischerweise werden bei dem erfindungsgemäßen Verfahren zur katalytischen Dehydrierung pro Durchgang durch den Reaktor (per-pass) Ausbeuten von 40 bis 80 %, bevorzugt von 50 bis 75 %, besonders bevorzugt von 60 bis 70 % bezogen auf den eingesetzten Kohlenwasserstoff erreicht. Insbesondere werden bei der katalytischen Dehydrierung von Ethylbenzol pro Durchgang durch den Reaktor Styrol-Ausbeuten von 40 bis 80 %, bevorzugt von 50 bis 75 %, besonders bevorzugt von 60 bis 70 % bezogen auf das eingesetzten Ethylbenzol, erreicht. Die angegebenen Ausbeuten beziehen sich auf Mol-%.

Typischerweise wird das Verfahren zur katalytischen Dehydrierung eines Kohlenwasserstoffs bei Temperaturen von 500 bis 650 °C und Drücken von 0,2 bis 2 bar absolut durchgeführt.

Weiterhin bezieht sich die vorliegende Erfindung auf die Verwendung eines oben beschriebenen Dehydrier-Katalysators zur katalytischen Dehydrierung eines Kohlenwasserstoffs, insbesondere eines alkylaromatischen oder aliphatischen Kohlenwasserstoffen, bevorzugt eines alkylaromatischen Kohlenwasserstoffs. Bevorzugt bezieht sich die Erfindung auf die Verwendung eines oben beschriebenen Dehydrier-Katalysators zur katalytischen Dehydrierung eines Kohlenwasserstoffs bei einem molaren Dampf/Kohlenwasserstoff-Verhältnis im Bereich von 3 bis 7,35; bevorzugt im Bereich von 4 bis 7; insbesondere bevorzugt im Bereich von 5 bis 6.

Weiterhin beschreibt die vorliegende Erfindung die Verwendung eines Dehydrier-Katalysators enthaltend mindestens eine Eisenverbindung, mindestens eine Kaliumverbindung, mindestens eine Cerverbindung und 0,7 bis 10 Gew.-% mindestens einer Manganverbindung, gerechnet als $MnO_2$,

zur katalytischen Dehydrierung eines Kohlenwasserstoffs, insbesondere eines alkylaromatischen oder aliphatischen Kohlenwasserstoffen, bevorzugt eines alkylaromatischen Kohlenwasserstoffs, besonders bevorzugt von Ethylbenzol, bei einem molaren Dampf/Kohlenwasserstoff-Verhältnis im Bereich von 3 bis 7,35; bevorzugt im Bereich von 4 bis 7; insbesondere bevorzugt im Bereich von 5 bis 6.

Im Folgenden werden die Figuren erläutert:

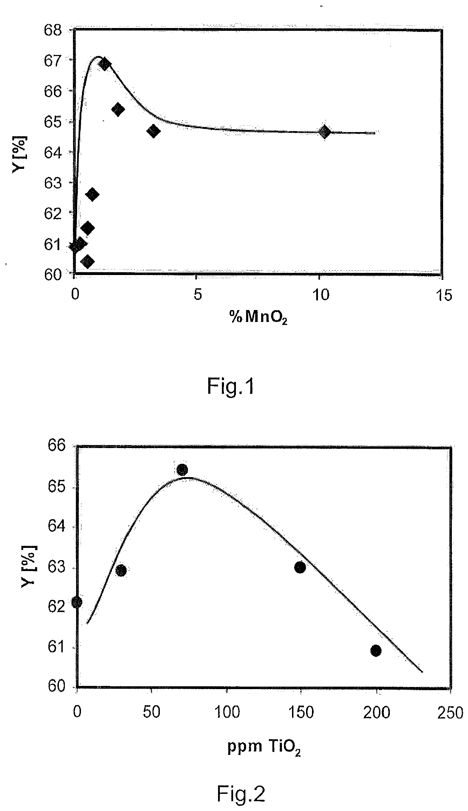

Figur 1 zeigt die Styrol-Ausbeute Y in Mol-% bei der katalytischen Dehydrierung von Ethylbenzol (gemäß Beispiel 7) bei einem Wasserdampf/Ethylbenzol-Gewichtsverhältnis von 1, einer Temperatur von 620 °C und einer Raumgeschwindigkeit von 1,26 ml Ethylbenzol/[(ml Katalysator)·(h)] in Abhängigkeit des Gehalts an Mangan in Gew.-% als $MnO_2$ im eingesetzten Katalysator (bezogen auf den gesamten Katalysator) und bei einem Titan-Gehalt im Katalysator von 70 ppm.

Figur 2 zeigt die Styrol-Ausbeute Y in Mol-% bei der katalytischen Dehydrierung von Ethylbenzol (gemäß Beispiel 7) bei einem Wasserdampf/Ethylbenzol-Gewichtsverhältnis von 1, einer Temperatur von 620 °C und einer Raumgeschwindigkeit von 1,26 ml Ethylbenzol/[(ml Katalysator)·(h)] in Abhängigkeit des Gehalts an Titan in ppm im eingesetzten Katalysator (bezogen auf den gesamten Katalysator) und bei einem Mangan-Gehalt im Katalysator von 1,8 Gew.-%.

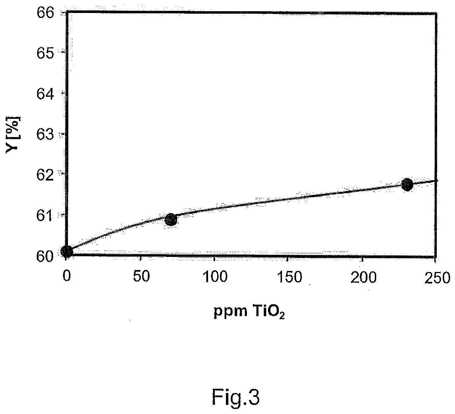

Figur 3 zeigt die Styrol-Ausbeute Y in Mol-% bei der katalytischen Dehydrierung von Ethylbenzol (gemäß Beispiel 7) bei einem Wasserdampf/Ethylbenzol-Gewichtsverhältnis von 1, einer Temperatur von 620 °C und einer Raumgeschwindigkeit von 1,26 ml Ethylbenzol/[(ml Katalysator)·(h)] in Abhängigkeit des Gehalts an Titan in ppm im eingesetzten Katalysator (bezogen auf den gesamten Katalysator) und bei einem Mangan-Gehalt im Katalysator von 0,02 Gew.-%.

Die Styrol-Ausbeute in Mol-% wird jeweils angegeben in Stoffmenge an hergestelltem Styrol bezogen auf Stoffmenge an eingesetztem Ethylbenzol.

Die vorliegende Erfindung soll durch die folgenden Beispiele näher erläutert werden.

Beispiele

Beispiel 1 (Vergleichsbeispiel) Katalysator A (ohne Zusatz von $MnO_2$ und $TiO_2$)

Es wurde ein Eisenoxid F1 (alpha-$Fe_2O_3$, Hämatit) eingesetzt, welches 0,027 Gew.-% Mangan (Mn), gerechnet als $MnO_2$, und 28 ppm Titan (Ti), gerechnet als $TiO_2$ enthielt. Die BET-Oberfläche des Eisenoxids F1 betrug 11 $m^2$/g.

Als weitere Komponenten wurden Kaliumcarbonat, Cercarbonat, Magnesiumoxid, Calciumhydroxid und Ammoniumheptamolybdat eingesetzt. Im Folgenden werden die mittels Elementaranalyse ermittelten Zusammensetzungen der eingesetzten Rohstoffe beschrieben, wobei sich die Angaben auf das jeweilige Element oder die jeweilige Verbindung bezogen auf den jeweiligen gesamten Rohstoff beziehen.

| | |
|---|---|
| Eisenoxid F1: | 98,9 Gew.-% $Fe_2O_3$; <10 ppm Chlor (Cl); 0,40 Gew.-% Schwefel (S); 170 ppm Mangan (Mn); 17 ppm Titan (Ti); 0,07 Gew.-% Chrom (Cr); 0,02 Gew.-% Calcium (Ca). |
| Kaliumcarbonat: | 85 Gew.-% $K_2CO_3$; <0,25 Gew.-% Natrium (Na); <20 ppm Chlor (Cl); <5 ppm Eisen (Fe). |
| Cercarbonat: | 52,80 Gew.-% $CeO_2$; 195 ppm Lanthan (La); 370 ppm Praseodym (Pr); 17 ppm Neodym (Nd); <10 ppm Titan (Ti); 3 ppm Chlor (Cl); 10 ppm Calcium (Ca); 0,52 Gew.-% Nitrat. |
| Magnesiumoxid: | 93,87 Gew.-% MgO; 1,40 Gew.-% Calcium (Ca); 0,50 Gew.-% Silicium (Si); 0,35 Gew.-% Eisen (Fe). |
| Calciumhydroxid: | 72,65 Gew.-% CaO; 0,35 Gew.-% Magnesium (Mg). |
| Ammoniumheptamolybdat: | 82 Gew.-% $MoO_3$; 90 ppm Kalium (K); 50 ppm Natrium (Na). |

Es wurde ein Katalysator A mit der nominalen Oxid-Zusammensetzung 72,7 Gew.-% $Fe_2O_3$, 13,6 Gew.-% $K_2O$, 7,4 Gew.-% $CeO_2$, 2,2 Gew.-% MgO, 2 Gew.-% CaO und 2,1 Gew.-% $MoO_3$, 0,02 Gew.-% $MnO_2$ und 20 ppm $TiO_2$ hergestellt.

Dazu wurden die oben genannten pulverförmigen Komponenten zunächst trocken vermischt und dann unter Zugabe von Wasser und Stärkelösung geknetet. Die Katalysator-Masse wurde extrudiert, wobei Pellets mit 3 mm Durchmesser erhalten wurden, und 1h bei 120°C getrocknet. Anschließend wurden die Katalysator-Formkörper (Pellets) 1 h bei 350°C und 1 h bei 825°C in Luft calciniert.

Beispiel 2 (Vergleichsbeispiel) Katalysator B (ohne Zusatz von $MnO_2$, mit Zugabe von $TiO_2$)

Es wurde ein Katalysator wie in Beispiel 1 beschrieben hergestellt, wobei aber im Gegensatz zum Beispiel 1 zusätzlich Titandioxid ($TiO_2$) zugegeben wurde. Es wurde das Eisenoxid F1 verwendet.

Es wurde ein Katalysator B mit der nominalen Oxid-Zusammensetzung von 72,7 Gew.-% $Fe_2O_3$, 13,6 Gew.-% $K_2O$, 7,4 Gew.-% $CeO_2$, 2,2 Gew.-% MgO, 2 Gew.-% CaO, 2,1 Gew.-% $MoO_3$, 0,02 Gew.-% $MnO_2$ und 70 ppm (mg/kg) $TiO_2$ erhalten.

Beispiel 3 (Vergleichsbeispiel) Katalysator C (Eisenoxid mit Anteil an Ti)

Es wurde ein Katalysator C ohne Zusatz von $MnO_2$ und $TiO_2$ hergestellt, wobei ein Eisenoxid F2 ($Fe_2O_3$, Hämatit) verwendet wurde, welches 0,025 Gew.-% Mangan (Mn), gerechnet als $MnO_2$, und einen Anteil an Titan von 195 ppm (mg/kg) (entspricht 325 ppm Ti, gerechnet als $TiO_2$) aufwies. Die BET-Oberfläche des Eisenoxids F2 betrug 2,3 $m^2$/g.

Die Zusammensetzung des Eisenoxids F2 wurde mittels Elementaranalyse bestimmt und ist im Folgenden wiedergegeben, wobei sich die Angaben auf das jeweilige Element oder Verbindung bezogen auf den gesamten Rohstoff beziehen.

Eisenoxid F2:     99,4 Gew.-% $Fe_2O_3$; <10 ppm Chlor (Cl); 0,16 Gew.-% Schwefel (S); 160 ppm Mangan (Mn); 195 ppm Titan (Ti); 0,02 Gew.-% Chrom (Cr).

Die Herstellung erfolgte wie im Beispiel 1 beschrieben und unter Verwendung von weiteren in Beispiel 1 beschriebenen Rohstoffe.

Es wurde ein Katalysator C mit der nominalen Oxid-Zusammensetzung 72,7 Gew.-% $Fe_2O_3$, 13,6 Gew.-% $K_2O$, 7,4 Gew.-% $CeO_2$, 2,2 Gew.-% MgO, 2 Gew.-% CaO, 2,1 Gew.-% $MoO_3$, 0,02 Gew.-% $MnO_2$ und 240 ppm $TiO_2$ erhalten.

Beispiel 4 Katalysatoren D-J (unterschiedliche Zugabe an Mangan)

Es wurde eine Reihe an Katalysatoren D, E, F, G, H, I, J mit Zusatz von verschiedenen Mengen von $MnO_2$ hergestellt. Der Titan-Gehalt wurde bei diesen Katalysatoren konstant auf 70 ppm eingestellt. Hierbei wurde ein Eisenoxid F3 eingesetzt ($Fe_2O_3$, Hämatit), welches 0,27 Gew.-% Mn (gerechnet als $MnO_2$) und Spuren Ti (<17 ppm als $TiO_2$) beinhaltete. Die BET-Oberfläche des Eisenoxids F3 betrug 1,2 $m^2/g$.

Weiterhin wurden Kaliumcarbonat, Cercarbonat, Magnesiumoxid, Calciumhydroxid, Ammoniumheptamolybdat, Mangandioxid und Titandioxid in solchen Mengen eingesetzt, dass Katalysatoren mit den tatsächlichen Oxid-Zusammensetzungen wie in Tabelle 1 dargestellt erhalten wurden. Soweit nicht anders angegeben wurden die in Beispiel 1 beschriebenen Rohstoffe verwendet.

Die Zusammensetzungen des Eisenoxids F3 und des eingesetzten Mangandioxids wurden mittels Elementaranalyse bestimmt und sind im Folgenden wiedergegeben, wobei sich die Angaben auf das jeweilige Element oder die jeweilige Verbindung bezogen auf den jeweiligen gesamten Rohstoff beziehen.

Eisenoxid F3:     99,6 Gew.-% $Fe_2O_3$; 280 ppm Chlor (Cl); <0,01 Gew.-% Schwefel (S); 0,17 Gew.-% Mangan (Mn); < 10 ppm Titan (Ti); < 10 ppm Chrom (Cr); < 10 ppm Calcium (Ca); 24 ppm Kupfer (Cu); 50 ppm Natrium (Na); 55 ppm Nickel (Ni); 43 ppm Silicium (Si); 16 ppm Zink (Zn).

Mangandioxid:     99,10 Gew.-% $MnO_2$; 0,14 Gew.-% Eisen (Fe).

Die Herstellung der Katalysator-Formkörper erfolgte wie im Beispiel 1 beschrieben.

Beispiel 5: Katalysatoren K-N (unterschiedliche Zugabe an Titan)

Es wurde eine Reihe an Katalysatoren K, L, M, N mit Zusatz von verschiedenen Mengen von $TiO_2$ hergestellt. Der Mangan-Gehalt wurde bei diesen Katalysatoren konstant auf 1,8 Gew.-% (gerechnet als $MnO_2$) eingestellt. Hierbei wurde das in Beispiel 4 beschriebenen Eisenoxid F3 eingesetzt ($Fe_2O_3$, Hämatit), welches 0,27 Gew.-% Mn (gerechnet als $MnO_2$) und kein Ti (<17 ppm gerechnet als $TiO_2$) enthält. Weiterhin wurden Kaliumcarbonat, Cercarbonat, Magnesiumoxid, Calciumhydroxid, Ammoniumheptamolybdat, Mangandioxid und Titandioxid in solchen Mengen eingesetzt, dass Katalysatoren mit der tatsächlichen Oxid-Zusammensetzungen wie in Tabelle 1 dargestellt erhalten wurden.

Die Herstellung der Katalysator-Formkörper erfolgte wie im Beispiel 1 beschrieben. Soweit nicht anders angegeben wurden die in Beispiel 1 beschriebenen Rohstoffe verwendet.

Beispiel 6: Katalysator O

Es wurde ein Katalysator O mit Zusatz von 70 ppm $TiO_2$ und Verwendung eines Eisenoxids F4, welches kein Titan (<17 ppm als $TiO_2$) und 0,6 Gew.-% Mn (gerechnet als $MnO_2$) enthält, hergestellt. Die BET-Oberfläche des Eisenoxids F4 betrug 1,1 $m^2/g$. Die Herstellung erfolgte wie beim Beispiel 2 beschrieben, unter Zusatz von $TiO_2$ als Titan-Quelle. Es wurden die weiteren in Beispiel 1 beschriebenen Rohstoffe verwendet.

Die Zusammensetzung des Eisenoxids F4 wurde mittels Elementaranalyse bestimmt und ist im Folgenden wiedergegeben, wobei sich die Angaben auf das jeweilige Element oder die jeweilige Verbindung bezogen auf den gesamten Rohstoff beziehen.

Eisenoxid F4:    99,4 Gew.-% $Fe_2O_3$; 63 ppm Chlor (Cl); <0,01 Gew.-% Schwefel (S); 0,39 Gew.-% Mangan (Mn); < 10 ppm Titan (Ti); < 10 ppm Chrom (Cr); < 10 ppm Calcium (Ca); 30 ppm Kupfer (Cu); 40 ppm Natrium (Na); 100 ppm Nickel (Ni); 36 ppm Silicium (Si); 40 ppm Zink (Zn).

Weiterhin wurden Kaliumcarbonat, Cercarbonat, Magnesiumoxid, Calciumhydroxid, Ammoniumheptamolybdat und Titandioxid in solchen Mengen eingesetzt, dass ein Katalysator mit der nominalen Oxid-Zusammensetzung 72,7% $Fe_2O_3$, 13,6% $K_2O$, 7,4% $CeO_2$, 2,2% MgO, 2% CaO, 2,1% $MoO_3$, 0,5 Gew.-% $MnO_2$ und 70 ppm $TiO_2$ erhalten wurde. Es wurde, soweit nicht anders angegeben, die Rohstoffe aus Beispiel 1 verwendet.

Die Zusammensetzungen der Katalysatoren wurden mittels Elementaranalyse überprüft und sind in Tabelle 1 zusammengestellt.

Tabelle 1: Zusammensetzungen aller Katalysatoren (Gew.-% als Oxid).

| Katalysator | $Fe_2O_3$ [Gew. -%] | $K_2O$ [Gew. -%] | $CeO_2$ [Gew. -%] | MgO [Gew. -%] | CaO [Gew. -%] | $MoO_3$ [Gew. -%] | $MnO_2$ [Gew. -%] | $TiO_2$ ppm |
|---|---|---|---|---|---|---|---|---|
| A | 72,7 | 13,6 | 7,4 | 2,2 | 2 | 2,1 | 0,02 | 20 |
| B | 72,7 | 13,6 | 7,4 | 2,2 | 2 | 2,1 | 0,02 | 70 |
| C | 72,7 | 13,6 | 7,4 | 2,2 | 2 | 2,1 | 0,02 | 240 |
| D | 72,5 | 13,6 | 7,4 | 2,2 | 2 | 2,1 | 0,2 | 70 |
| E | 72,2 | 13,6 | 7,4 | 2,2 | 2 | 2,1 | 0,5 | 70 |
| F | 72,0 | 13,6 | 7,4 | 2,2 | 2 | 2,1 | 0,7 | 70 |
| G | 71,5 | 13,6 | 7,4 | 2,2 | 2 | 2,1 | 1,2 | 70 |
| H | 71,2 | 13,6 | 7,4 | 2,2 | 2 | 2,1 | 1,8 | 70 |
| I | 69,5 | 13,6 | 7,4 | 2,2 | 2 | 2,1 | 3,2 | 70 |
| J | 62,5 | 13,6 | 7,4 | 2,2 | 2 | 2,1 | 10,2 | 70 |
| K | 71,2 | 13,6 | 7,4 | 2,2 | 2 | 2,1 | 1,8 | 0 |
| L | 71,2 | 13,6 | 7,4 | 2,2 | 2 | 2,1 | 1,8 | 30 |
| M | 71,2 | 13,6 | 7,4 | 2,2 | 2 | 2,1 | 1,8 | 150 |
| N | 71,2 | 13,6 | 7,4 | 2,2 | 2 | 2,1 | 1,8 | 200 |
| O | 72,2 | 13,6 | 7,4 | 2,2 | 2 | 2,1 | 0,5 | 70 |

Beispiel 7: Dehydrierung von Ethylbenzol zu Styrol bei D/KW von 1,0

Die Katalysatoren A bis O aus den Beispielen 1 bis 6 wurden in der Dehydrierung von Ethylbenzol zu Styrol in Anwesenheit von Wasserdampf eingesetzt. In einem isothermen Rohrreaktor wurden 13,3 mL Katalysator eingebaut. Bei 620°C und 1 atm Ausgangsdruck wurde der Katalysator mit 14,6 g/h Ethylbenzol und 14,6 g/h vollentsalztem (VE) Wasser, entsprechend einem D/KW-Gewichtsverhältnis von 1,0, kontinuierlich beaufschlagt. Nach Stabilisierung (nach ca. 40 h) wurde die Ausbeute an Styrol gaschromatographisch bestimmt. Die Ergebnisse zu Ethylbenzol-Umsatz, Styrol-Selektivität und -Ausbeute der verschiedenen Katalysatoren sind in der Tabelle 2 und in den Figuren 1 und 2 dargestellt.

Ethylbenzol-Umsatz, Styrol-Selektivität und -Ausbeute wurden anhand der folgenden Formeln bestimmt:

$$\text{Umsatz (Mol-\%)} = [A*M_f - B*M_p)/(A*M_f)] \times 100$$

$$\text{Selektivität (Mol-\%)} = [D*M_p - C*M_f)/(A*M_f - B*M_p)] \times ( M_{EB}/M_{ST}) \times 100$$

$$\text{Ausbeute (Mol-\%)= Umsatz x Selektivität / 100}$$

mit:

A: Ethylbenzol-Konzentration am Reaktor-Eingang (Gew.-%)
B: Ethylbenzol-Konzentration am Reaktor-Ausgang (Gew.-%)
C: Styrol-Konzentration am Reaktor-Eingang (Gew.-%)
D: Styrol-Konzentration am Reaktor-Ausgang (Gew.-%)
$M_f$: Mittlere Molmasse der organischen Edukte
$M_p$: Mittlere Molmasse der organischen Produkte
$M_{EB}$: Molmasse Ethylbenzol
$M_{ST}$: Molmasse Styrol

Die oben genannten Angaben bezüglich Konzentration und Molmassen beziehen sich jeweils auf die organische Phase (ohne Wasser).

Figur 1 stellt die Abhängigkeit der Styrol-Ausbeute in Abhängigkeit des Anteils an Mangan (Mn) im verwendeten Dehydrier-Katalysator dar. Die Werte beziehen sich auf Katalysatoren, die jeweils einen konstanten Anteil an Titan von 70 ppm (gerechnet als $TiO_2$) aufweisen, und auf ein D/KW-Gewichtsverhältnis bei der Dehydrierung von 1. Man erkennt deutlich, dass sich eine verbesserte Ausbeute ab einem Mangan-Anteil im Katalysator von mindestens 0,7 Gew.-% ergibt. Eine besonders hohe Ausbeute an Styrol kann mit Katalysatoren mit einem Mangangehalt im Bereich von 0,7 bis 10 Gew.-%, insbesondere von 0,7 bis 5 Gew.-% erzielt werden.

Figur 2 stellt die Abhängigkeit der Styrol-Ausbeute in Abhängigkeit des Anteils an Titan (Ti) im verwendeten Dehydrier-Katalysator dar. Die Werte beziehen sich auf Katalysatoren, die jeweils einen konstanten Anteil an Mangan von insgesamt 1,8 Gew.-% (berechnet als $MnO_2$) aufweisen, und auf ein D/KW-Gewichtsverhältnis bei der Dehydrierung von 1. Man erkennt deutlich, dass sich eine optimierte Styrol-Ausbeute bei einem Titan-Anteil im Bereich von 10 bis 200 ppm, insbesondere von 30 bis 150 ppm, insbesondere von 50 bis 100 ppm ergibt.

Figur 3 stellt die Abhängigkeit der Styrol-Ausbeute in Abhängigkeit des Anteils an Titan (Ti) im verwendeten Dehydrier-Katalysator dar. Die Werte beziehen sich auf Katalysatoren, die jeweils einen konstanten Anteil an Mangan von insgesamt 0,02 Gew.-% (berechnet als $MnO_2$) aufweisen, und auf ein D/KW-Gewichtsverhältnis bei der Dehydrierung von 1. Es konnte gezeigt werden, dass bei einem D/KW-Verhältnis von 1 und bei Katalysatoren mit einem geringen Mangan-Gehalt nur eine wesentlich geringere Erhöhung der Ausbeute durch Zugabe von Titan erzielt werden kann. Dagegen lässt sich der positive Einfluss des Mangan-Anteils von mindestens 0,7 Gew.-% durch eine gezielte Auswahl des Titan-Gehaltes noch einmal verbessern.

Tabelle 2: Ergebnisse zur Dehydrierung von Ethylbenzol bei einem D/KW-Gewichtsverhältnis von 1 und 620°C.

| Katalysator | Mn Zugabe | Mn Ist | $TiO_2$ | Umsatz Ethylbenzol (EB) | Selektivität Styrol | Ausbeute Styrol |
|---|---|---|---|---|---|---|
| | % Oxid | % Oxid | ppm | Mol-% EB | Mol-% ST | Mol-% ST |
| A | 0 | 0,02 | 0 | 62,7 | 95,9 | 60,1 |
| B | 0 | 0,02 | 70 | 63,8 | 95,5 | 60,9 |
| C | 0 | 0,02 | 240 | 64,0 | 96,5 | 61,8 |
| D | 0 | 0,2 | 70 | 63,6 | 95,9 | 61,0 |
| E | 0,3 | 0,5 | 70 | 64,1 | 95,9 | 61,5 |
| F | 0,5 | 0,7 | 70 | 65,3 | 95,9 | 62,6 |
| G | 1,0 | 1,2 | 70 | 70,2 | 95,2 | 66,9 |
| H | 1,6 | 1,8 | 70 | 68,7 | 95,2 | 65,4 |
| I | 3 | 3,2 | 70 | 67,8 | 95,4 | 64,7 |
| J | 10 | 10,2 | 70 | 67,7 | 95,6 | 64,7 |
| K | 1,6 | 1,8 | 0 | 64,9 | 95,6 | 62,1 |
| L | 1,6 | 1,8 | 30 | 65,8 | 95,6 | 62,9 |
| M | 1,6 | 1,8 | 150 | 65,7 | 95,9 | 63,0 |

(fortgesetzt)

| Katalysator | Mn Zugabe | Mn Ist | TiO$_2$ | Umsatz Ethylbenzol (EB) | Selektivität Styrol | Ausbeute Styrol |
|---|---|---|---|---|---|---|
| | % Oxid | % Oxid | ppm | Mol-% EB | Mol-% ST | Mol-% ST |
| N | 1,6 | 1,8 | 200 | 63,3 | 96,1 | 60,9 |
| O | 0 | 0,50 | 70 | 62,8 | 96,2 | 60,4 |

Beispiel 8: Dehydrierung von Ethylbenzol zu Styrol bei D/KW von 1,25

Die Katalysatoren A bis O von den Beispielen 1 bis 6 wurden in der Dehydrierung von Ethylbenzol zu Styrol in Anwesenheit von Wasserdampf wie in Beispiel 7 beschrieben eingesetzt, wobei ein D/KW-Gewichtsverhältnis von 1,25 eingestellt wurde. In einem isothermen Rohrreaktor wurden 13,3 mL Katalysator eingebaut. Bei 620°C und 1 atm Ausgangsdruck wurde der Katalysator mit 14,6 g/h Ethylbenzol und 18,25 g/h vollentsalztem (VE) Wasser, entsprechend einem D/KW-Gewichtsverhältnis von 1,25, kontinuierlich beaufschlagt.

Die Ergebnisse zu Ethylbenzol-Umsatz, Styrol-Selektivität und -Ausbeute der verschiedenen Katalysatoren sind in der Tabelle 3 dargestellt. In Tabelle 3 sind zudem die Abnahmen der Styrol-Ausbeuten beim Übergang von einem D/KW-Gewichtsverhältnis von 1,25 zu einem D/KW-Gewichtsverhältnis von 1,0 dargestellt (Delta Y 1,25→1,0).

Tabelle 3: Ergebnisse zur Dehydrierung von Ethylbenzol bei einem D/KW-Gewichtsverhältnis von 1,25 und 620° C.

| Katalysator | Mn Zugabe | Mn Ist | TiO$_2$ | Umsatz Ethylbenzol (EB) | Selektivität Styrol | Ausbeute Styrol | Delta Y 1,25→1,0 |
|---|---|---|---|---|---|---|---|
| | % Oxid | % Oxid | ppm | Mol-% EB | Mol-% ST | Mol-% ST | % |
| A | 0 | 0,02 | 0 | 71,4 | 95,4 | 68,1 | 8,0 |
| B | 0 | 0,02 | 70 | 70,4 | 96,1 | 67,6 | 6,7 |
| C | 0 | 0,02 | 240 | 73,5 | 95,7 | 70,3 | 8,5 |
| D | 0 | 0,2 | 70 | 68,5 | 95,7 | 65,6 | 4,6 |
| E | 0,3 | 0,5 | 70 | 72,9 | 95,5 | 69,6 | 8,1 |
| F | 0,5 | 0,7 | 70 | 71,6 | 95,6 | 68,5 | 5,9 |
| G | 1,0 | 1,2 | 70 | 73,1 | 95,3 | 69,7 | 2,8 |
| H | 1,6 | 1,8 | 70 | 73,2 | 95,0 | 69,5 | 4,1 |
| I | 3 | 3,2 | 70 | 72,7 | 95,2 | 69,2 | 4,5 |
| J | 10 | 10,2 | 70 | 72,1 | 95,6 | 68,9 | 4,2 |
| K | 1,6 | 1,8 | 0 | 70,0 | 95,4 | 66,8 | 4,7 |
| L | 1,6 | 1,8 | 30 | 70,8 | 95,5 | 67,6 | 4,7 |
| M | 1,6 | 1,8 | 150 | 72,3 | 95,6 | 69,1 | 6,1 |
| N | 1,6 | 1,8 | 200 | 71,7 | 97,2 | 69,7 | 8,8 |
| O | 0 | 0,50 | 70 | 71,0 | 95,9 | 68,1 | 7,7 |

Die bessere Stabilität des Katalysators zeigte sich zudem durch einen geringeren Verlust an Katalysatoraktivität, wenn man von einem mittleren zu einem geringen D/KW-Verhältnis übergeht (Vergleich der Ergebnisse der Beispiele 7 und 8)

Beispiel 9: Dehydrierung von Buten zu Butadien

Der Katalysator H wurde in der Dehydrierung von 1-Buten zu Butadien eingesetzt. Ein Volumen von 38 mL Katalysator wurde in einem isotherm beheizten Rohrreaktor eingesetzt. Bei 620 °C und 1 atm wurde der Reaktor mit 60 g/h vollentsalztem Wasser und 22,5 g/h 1-Buten (entsprechend einem Molverhältnis von Wasserdampf/Buten von 8,6) kontinuierlich beaufschlagt. Nach Stabilisierung (etwa 16 h) wurde das erhaltene Produktgemisch gaschromatographisch analysiert. Der Umsatz an 1-Buten und die Butadien-Selektivität wurden mit den Formeln wie in Beispiel 7 berechnet, wobei 1-Buten an die Stelle von Ethylbenzol und Butadien an die Stelle von Styrol zu setzen sind. Der Buten-Umsatz betrug 23,3 Mol-% und die Butadien-Selektivität 91,2 Mol-%.

**Patentansprüche**

1. Dehydrier-Katalysator enthaltend mindestens eine Eisenverbindung, mindestens eine Kaliumverbindung, mindestens eine Cerverbindung, 0,1 bis 10 Gew.-% mindestens einer Calciumverbindung, gerechnet als CaO, 0,7 bis 10 Gew.-% mindestens einer Manganverbindung, gerechnet als $MnO_2$, und 30 bis 150 ppm mindestens einer Titanverbindung, gerechnet als $TiO_2$.

2. Dehydrier-Katalysator gemäß Anspruch 1, **dadurch gekennzeichnet, dass** der Katalysator 0,7 bis 3 Gew.-% mindestens einer Manganverbindung, gerechnet als $MnO_2$, enthält.

3. Dehydrier-Katalysator gemäß einem der Ansprüche 1 bis 2, **dadurch gekennzeichnet, dass** der Katalysator

   50 bis 90 Gew.-% mindestens einer Eisenverbindung, gerechnet als $Fe_2O_3$;
   1 bis 30 Gew.-% mindestens einer Kaliumverbindung, gerechnet als $K_2O$;
   0,7 Gew.-% bis 10 Gew.-% mindestens einer Manganverbindung, gerechnet als $MnO_2$;
   30 bis 150 ppm mindestens einer Titanverbindung, gerechnet als $TiO_2$;
   2 bis 20 Gew.-% mindestens einer Cerverbindung, gerechnet als $CeO_2$; und
   optional 0 bis 30 Gew.-% mindestens einer weiteren Komponente
   enthält.

4. Dehydrier-Katalysator gemäß einem der Ansprüche 1 bis 3, **dadurch gekennzeichnet, dass** der Katalysator als weitere Komponente 0,1 bis 10 Gew.-% mindestens einer Verbindung ausgewählt aus der Gruppe bestehend aus Molybdän, Wolfram und Vanadium, gerechnet als Oxid in der jeweils höchsten Oxidationsstufe, enthält.

5. Dehydrier-Katalysator gemäß einem der Ansprüche 1 bis 4, **dadurch gekennzeichnet, dass** der Katalysator

   50 bis 90 Gew.-% mindestens einer Eisenverbindung, gerechnet als $Fe_2O_3$;
   1 bis 30 Gew.-% mindestens einer Kaliumverbindung, gerechnet als $K_2O$;
   0,7 bis 10 Gew.-% mindestens einer Manganverbindung, gerechnet als $MnO_2$;
   30 bis 150 ppm mindestens einer Titanverbindung, gerechnet als $TiO_2$;
   2 bis 20 Gew.-% mindestens einer Cerverbindung, gerechnet als $CeO_2$;
   0,1 bis 10 Gew.-% mindestens einer Magnesiumverbindung, gerechnet als MgO;
   0,1 bis 10 Gew.-% mindestens einer Calciumverbindung, gerechnet als CaO;
   0,1 bis 10 Gew.-% mindestens einer Molybdänverbindung, gerechnet als $MoO_3$;
   0 bis 10 Gew.-% mindestens einer Vanadiumverbindung, gerechnet als $V_2O_5$ und
   0 bis 10 Gew.-% mindestens einer weiteren Komponente
   enthält.

6. Verfahren zur Herstellung eines Dehydrier-Katalysators gemäß einem der Anspruche 1 bis 5, umfassend die folgenden Schritte

   i) Herstellen einer Katalysator-Vormischung durch Vermischen mindestens einer Eisenverbindung, mindestens einer Kaliumverbindung, 0,1 bis 10 Gew.-%, bezogen auf den fertigen Katalysator, mindestens einer Calciumverbindung, gerechnet als CaO, mindestens einer Cerverbindung, 0,7 bis 10 Gew.-%, bezogen auf den fertigen Katalysator, mindestens einer Manganverbindung, gerechnet als $MnO_2$, 30 bis 150 ppm, bezogen auf den fertigen Katalysator, mindestens einer Titanverbindung, gerechnet als $TiO_2$, optional weiterer Metallverbindungen, optional weiterer Komponenten und optional mindestens eines Bindemittels mit einem Lösemittel;
   ii) Herstellen von Katalysator-Formkörpern aus der in Schritt i) erhaltenen Katalysator-Vormischung;
   iii) Trocknen der Katalysator-Formkörper und Kalzinieren der Katalysator-Formkörper.

7. Verfahren zur Herstellung eines Dehydrier-Katalysators gemäß Anspruch 6, **dadurch gekennzeichnet dass** die Katalysator-Formkörpern in Schritt iii) bei Temperaturen im Bereich von 500 bis 1200 °C kalziniert werden.

8. Verfahren zur katalytischen Dehydrierung eines Kohlenwasserstoffs, wobei ein Gemisch aus Wasserdampf und mindestens einem Kohlenwasserstoff mit einem Dehydrier-Katalysator gemäß einem der Ansprüche 1 bis 5 in Kontakt gebracht wird.

9. Verfahren zur katalytischen Dehydrierung eines Kohlenwasserstoffs gemäß Anspruch 8, **dadurch gekennzeichnet, dass** ein Gemisch aus Wasserdampf und mindestens einem Kohlenwasserstoff mit einem molaren Dampf/Kohlenwasserstoff-Verhältnis im Bereich von 3 bis 7,35 eingesetzt wird.

10. Verfahren zur katalytischen Dehydrierung gemäß einem der Anspruch 8 oder 9, **dadurch gekennzeichnet, dass** ein Gemisch aus Wasserdampf und mindestens einem Kohlenwasserstoff mit einem molaren Dampf/Kohlenwasserstoff-Verhältnis im Bereich von 4 bis 7 eingesetzt wird.

11. Verfahren zur katalytischen Dehydrierung gemäß einem der Ansprüche 8 bis 10, **dadurch gekennzeichnet, dass** es sich bei dem Kohlenwasserstoff um Ethylbenzol handelt.

12. Verwendung eines Dehydrier-Katalysators gemäß einem der Ansprüche 1 bis 5 zur katalytischen Dehydrierung eines Kohlenwasserstoffs.

13. Verwendung eines Dehydrier-Katalysators gemäß Anspruch 12 zur katalytischen Dehydrierung eines Kohlenwasserstoffs bei einem molaren Dampf/Kohlenwasserstoff-Verhältnis im Bereich von 3 bis 7,35.

**Claims**

1. A dehydrogenation catalyst comprising at least one iron compound, at least one potassium compound, at least one cerium compound, from 0.1 to 10% by weight of at least one calcium compound calculated as CaO, from 0.7 to 10% by weight of at least one manganese compound, calculated as $MnO_2$, and from 30 to 150 ppm of at least one titanium compound, calculated as $TiO_2$.

2. The dehydrogenation catalyst according to claim 1, wherein the catalyst comprises from 0.7 to 3% by weight of at least one manganese compound, calculated as $MnO_2$.

3. The dehydrogenation catalyst according to either claim 1 or 2, wherein the catalyst comprises

   from 50 to 90% by weight of at least one iron compound, calculated as $Fe_2O_3$;
   from 1 to 30% by weight of at least one potassium compound, calculated as $K_2O$;
   from 0.7 to 10% by weight of at least one manganese compound, calculated as $MnO_2$;
   from 30 to 150 ppm of at least one titanium compound, calculated as $TiO_2$;
   from 2 to 20% by weight of at least one cerium compound, calculated as $CeO_2$; and
   optionally from 0 to 30% by weight of at least one further component.

4. The dehydrogenation catalyst according to any of claims 1 to 3, wherein the catalyst comprises from 0.1 to 10% by weight of at least one compound selected from the group consisting of molybdenum, tungsten and vanadium, calculated as oxide in the respective highest oxidation state, as further component.

5. The dehydrogenation catalyst according to any of claims 1 to 4, wherein the catalyst comprises

   from 50 to 90% by weight of at least one iron compound, calculated as $Fe_2O_3$;
   from 1 to 30% by weight of at least one potassium compound, calculated as $K_2O$;
   from 0.7 to 10% by weight of at least one manganese compound, calculated as $MnO_2$;
   from 30 to 150 ppm of at least one titanium compound, calculated as $TiO_2$;
   from 2 to 20% by weight of at least one cerium compound, calculated as $CeO_2$;
   from 0.1 to 10% by weight of at least one magnesium compound, calculated as MgO;
   from 0.1 to 10% by weight of at least one calcium compound, calculated as CaO;
   from 0.1 to 10% by weight of at least one molybdenum compound, calculated as $MoO_3$;
   from 0 to 10% by weight of at least one vanadium compound, calculated as $V_2O_5$, and
   from 0 to 10% by weight of at least one further component.

6. A process for producing a dehydrogenation catalyst according to any of claims 1 to 5, which comprises the following steps

    i) production of a catalyst premix by mixing at least one iron compound, at least one potassium compound, from 0.1 to 10% by weight, based on the finished catalyst, of at least one calcium compound, calculated as CaO, at least one cerium compound, from 0.7 to 10% by weight, based on the finished catalyst, of at least one manganese compound, calculated as $MnO_2$, from 30 to 150 ppm, based on the finished catalyst, of at least one titanium compound, calculated as $TiO_2$, optionally further metal compounds, optionally further components and optionally at least one binder with a solvent;
    ii) production of shaped catalyst bodies from the catalyst premix obtained in step i);
    iii) drying of the shaped catalyst bodies and calcination of the shaped catalyst bodies.

7. The process for producing a dehydrogenation catalyst according to claim 6, wherein the shaped catalyst bodies are calcined at temperatures in the range from 500 to 1200°C in step iii).

8. A process for the catalytic dehydrogenation of a hydrocarbon, wherein a mixture of steam and at least one hydrocarbon is brought into contact with a dehydrogenation catalyst according to any of claims 1 to 5.

9. The process for the catalytic dehydrogenation of a hydrocarbon according to claim 8, wherein a mixture of steam and at least one hydrocarbon having a molar steam/hydrocarbon ratio in the range from 3 to 7.35 is used.

10. The catalytic dehydrogenation process according to either claim 8 or 9, wherein a mixture of steam and at least one hydrocarbon having a molar steam/hydrocarbon ratio in the range from 4 to 7 is used.

11. The catalytic dehydrogenation process according to any of claims 8 to 10, wherein the hydrocarbon is ethylbenzene.

12. The use of a dehydrogenation catalyst according to any of claims 1 to 5 for the catalytic dehydrogenation of a hydrocarbon.

13. The use of a dehydrogenation catalyst according to claim 12 for the catalytic dehydrogenation of a hydrocarbon at a molar steam/hydrocarbon ratio in the range from 3 to 7.35.


**Revendications**

1. Catalyseur de déshydrogénation, contenant au moins un composé de fer, au moins un composé de potassium, au moins un composé de cérium, 0,1 à 10 % en poids d'au moins un composé de calcium, calculé en tant que CaO, 0,7 à 10 % en poids d'au moins un composé de manganèse, calculé en tant que $MnO_2$, et 30 à 150 ppm d'au moins un composé de titane, calculé en tant que $TiO_2$.

2. Catalyseur de déshydrogénation selon la revendication 1, **caractérisé en ce que** le catalyseur contient 0,7 à 3 % en poids d'au moins un composé de manganèse, calculé en tant que $MnO_2$.

3. Catalyseur de déshydrogénation selon l'une quelconque des revendications 1 à 2, **caractérisé en ce que** le catalyseur contient :

    50 à 90 % en poids d'au moins un composé de fer, calculé en tant que $Fe_2O_3$ ;
    1 à 30 % en poids d'au moins un composé de potassium, calculé en tant que $K_2O$ ;
    0,7 % en poids à 10 % en poids d'au moins un composé de manganèse, calculé en tant que $MnO_2$ ;
    30 à 150 ppm d'au moins un composé de titane, calculé en tant que $TiO_2$ ;
    2 à 20 % en poids d'au moins un composé de cérium, calculé en tant que $CeO_2$ ; et
    éventuellement 0 à 30 % en poids d'au moins un composant supplémentaire.

4. Catalyseur de déshydrogénation selon l'une quelconque des revendications 1 à 3, **caractérisé en ce que** le catalyseur contient en tant que composant supplémentaire 0,1 à 10 % en poids d'au moins un composé choisi dans le groupe constitué par le molybdène, le tungstène et le vanadium, calculé en tant qu'oxyde au niveau d'oxydation le plus élevé respectif.

**5.** Catalyseur de déshydrogénation selon l'une quelconque des revendications 1 à 4, **caractérisé en ce que** le catalyseur contient :

50 à 90 % en poids d'au moins un composé de fer, calculé en tant que $Fe_2O_3$ ;
1 à 30 % en poids d'au moins un composé de potassium, calculé en tant que $K_2O$ ;
0,7 à 10 % en poids d'au moins un composé de manganèse, calculé en tant que $MnO_2$ ;
30 à 150 ppm d'au moins un composé de titane, calculé en tant que $TiO_2$ ;
2 à 20 % en poids d'au moins un composé de cérium, calculé en tant que $CeO_2$ ;
0,1 à 10 % en poids d'au moins un composé de magnésium, calculé en tant que MgO ;
0,1 à 10 % en poids d'au moins un composé de calcium, calculé en tant que CaO ;
0,1 à 10 % en poids d'au moins un composé de molybdène, calculé en tant que $MoO_3$ ;
0 à 10 % en poids d'au moins un composé de vanadium, calculé en tant que $V_2O_5$ ; et
0 à 10 % en poids d'au moins un composant supplémentaire.

**6.** Procédé de fabrication d'un catalyseur de déshydrogénation selon l'une quelconque des revendications 1 à 5, comprenant les étapes suivantes :

i) la fabrication d'un mélange préliminaire de catalyseur par mélange d'au moins un composé de fer, d'au moins un composé de potassium, de 0,1 à 10 % en poids, par rapport au catalyseur fini, d'au moins un composé de calcium, calculé en tant que CaO, d'au moins un composé de cérium, de 0,7 à 10 % en poids, par rapport au catalyseur fini, d'au moins un composé de manganèse, calculé en tant que $MnO_2$, de 30 à 150 ppm, par rapport au catalyseur fini, d'au moins un composé de titane, calculé en tant que $TiO_2$, éventuellement de composés métalliques supplémentaires, éventuellement de composants supplémentaires et éventuellement d'au moins un liant avec un solvant ;
ii) la fabrication de corps moulés de catalyseur à partir du mélange préliminaire de catalyseur obtenu à l'étape i) ;
iii) le séchage des corps moulés de catalyseur et la calcination des corps moulés de catalyseur.

**7.** Procédé de fabrication d'un catalyseur de déshydrogénation selon la revendication 6, **caractérisé en ce que** les corps moulés de catalyseur sont calcinés à l'étape iii) à des températures dans la plage allant de 500 à 1 200 °C.

**8.** Procédé de déshydrogénation catalytique d'un hydrocarbure, selon lequel un mélange de vapeur d'eau et d'au moins un hydrocarbure est mis en contact avec un catalyseur de déshydrogénation selon l'une quelconque des revendications 1 à 5.

**9.** Procédé de déshydrogénation catalyseur d'un hydrocarbure selon la revendication 8, **caractérisé en ce qu'**un mélange de vapeur d'eau et d'au moins un hydrocarbure est utilisé en un rapport molaire vapeur/hydrocarbure dans la plage allant de 3 à 7,35.

**10.** Procédé de déshydrogénation catalytique selon l'une quelconque des revendications 8 ou 9, **caractérisé en ce qu'**un mélange de vapeur d'eau et d'au moins un hydrocarbure est utilisé en un rapport molaire vapeur/hydrocarbure dans la plage allant de 4 à 7.

**11.** Procédé de déshydrogénation catalytique selon l'une quelconque des revendications 8 à 10, **caractérisé en ce que** l'hydrocarbure est l'éthylbenzène.

**12.** Utilisation d'un catalyseur de déshydrogénation selon l'une quelconque des revendications 1 à 5 pour la déshydrogénation catalytique d'un hydrocarbure.

**13.** Utilisation d'un catalyseur de déshydrogénation selon la revendication 12 pour la déshydrogénation catalytique d'un hydrocarbure à un rapport molaire vapeur/hydrocarbure dans la plage allant de 3 à 7,35.

Fig.1

Fig.2

Fig.3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0181999 A
- WO 9618457 A
- EP 0956899 B
- EP 0502510 A
- US 4220560 A
- US 20060106267 A
- WO 9949966 A
- CN 1470325 A
- CN 1253855 A
- WO 2003064032 A

**In der Beschreibung aufgeführte Nicht-Patentliteratur**

- **MIYAKOSHI et al.** *Appl. Cat. A,* 2001, vol. 216, 137-146
- **KOTARBA et al.** *J. Cat.,* 2004, vol. 221, 650-652
- **LIAO et al.** *Cat. Comm.,* 2008, vol. 9, 1817-1821